(19) Europäisches Patentamt / European Patent Office / Office européen des brevets

(11) **EP 3 391 901 B1**

(12) **EUROPEAN PATENT SPECIFICATION**

(45) Date of publication and mention
of the grant of the patent:
**05.07.2023 Bulletin 2023/27**

(21) Application number: **16874926.5**

(22) Date of filing: **16.12.2016**

(51) International Patent Classification (IPC):
**A61K 38/48** (2006.01)     **A61P 39/00** (2006.01)

(52) Cooperative Patent Classification (CPC):
**A61K 38/484; A61K 38/48; A61K 38/49;**
**A61P 1/16;** C12Y 304/21007; C12Y 304/21068;
C12Y 304/21073; C12Y 304/23048; Y02A 50/30

(86) International application number:
**PCT/CN2016/110451**

(87) International publication number:
**WO 2017/101869 (22.06.2017 Gazette 2017/25)**

(54) **PLASMINOGEN FOR USE IN TREATING LIVER TISSUE DAMAGE**

PLASMINOGEN ZUR BEHANDLUNG VON LEBERGEWEBESCHÄDEN

LE PLASMINOGÈNE POUR TRAITER DES LÉSIONS TISSULAIRES HÉPATIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.12.2015 PCT/CN2015/097945
18.12.2015 PCT/CN2015/097949**

(43) Date of publication of application:
**24.10.2018 Bulletin 2018/43**

(73) Proprietor: **Talengen International Limited
Hongkong (CN)**

(72) Inventor: **LI, Jinan
Shenzhen
Guangdong518020 (CN)**

(74) Representative: **Abel & Imray LLP
Westpoint Building
James Street West
Bath BA1 2DA (GB)**

(56) References cited:
**WO-A2-2008/026999     CN-A- 1 768 138
CN-A- 101 628 113     CN-A- 103 656 630**

US-A1- 2003 147 876

- **J. MATHYS VOGTEN ET AL: "Angiostatin inhibits experimental liver fibrosis in mice",** INTERNATIONAL JOURNAL OF COLORECTAL DISEASE, vol. 19, no. 4, 10 January 2004 (2004-01-10), pages 387-394, XP055627236, DE ISSN: 0179-1958, DOI: **10.1007/s00384-003-0562-4**
- **LI, ZHEN et al.: "Advances in Studies on Treatment for Hepatic Fibrosis",** Journal of LIAONING MEDICAL UNIVERSITY, vol. 28, no. 2, 30 April 2007 (2007-04-30) , XP009511416,
- **LIU, MINGYING et al.: "Plasminogen: Structure, Function and Evolution",** JOURNAL OF OCEAN UNIVERSITY OF CHINA, vol. 40, no. 10, 31 October 2010 (2010-10-31), pages 69-74, XP009510823, CHINA ISSN: 1672-5174, DOI: **10.16441/j.cnki.hdxb.2010.10.012**
- **YU , DANFENG et al.: "Measurements of Plasmin-Alpha 2 Antiplasmin Complex in Patients with Liver Cirrhosis and Hepatocarcinoma",** LABORATORY MEDICINE AND CLINIC, vol. 6, no. 2, 31 January 2009 (2009-01-31), pages 92-93, XP009511290,

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 391 901 B1

## Description

## Technical Field

The present invention relates to the use of plasminogen or plasmin in the prevention and/or treatment of hepatic tissue injury caused by various reasons, thereby providing a brand new therapeutic strategy for treating hepatic tissue injury and its related disorders.

## Background Art

Hepatic injury or hepatic tissue injury is a hepatic parenchymal disease caused by various reasons, and is a general term for a series of pathological changes, such as hepatic tissue inflammation, hepatocyte degeneration, necrosis and hepatic tissue fibrosis. Common causes are inflammation, liver congestion, viral infections, poisoning, drugs, radiation, etc. Some diseases, such as diabetes mellitus, hepatitis, hypertension, and atherosclerosis, also occur with injuries of hepatic tissue cells.

Drugs are very common causes of hepatic tissue damage. Common drugs that cause hepatic tissue damage include: anti-tuberculosis drugs, such as rifampicin, isoniazid, and ethambutol; anti-tumor drugs, such as cyclophosphamide, methotrexate, 5-fluorouracil, carboplatin, and cisplatin; lipid-regulating and lipid-lowering drugs, such as statins (atorvastatin and lovastatin), fenofibrate, clofibrate, and niacin; steroid hormones, such as estrogenic drugs, oral contraceptives, and male anabolic hormones; cardiovascular drugs, such as amiodarone, warfarin, and calcium ion antagonists; anti-rheumatic drugs, such as antifan, fenbufen, aspirin, and indomethacin; antibiotics, such as chloramphenicol, roxithromycin, ketoconazole, penicillins, and sulfonamides; anti-allergic drugs, such as promethazine (Phenergan), chlorpheniramine (Chlorphenamine), and loratadine (Clarityne); anti-ulcer drugs, such as cimetidine, ranitidine, and famotidine; antifungal drugs, such as ribavirin; and so on.

Alcohol is a great threat to the liver. Long-term or intermittent heavy drinking can cause hepatic tissue injury. The greater the drinking amount of alcohol is and the longer the drinking history is, the more severe the consequences are. Alcohol directly poisons hepatocytes and affects their structures and functions.

Alcoholic hepatic injury is a chronic, toxic hepatic injury, a hepatic disease caused by long-term heavy drinking. In the early stage, it is usually manifested as fatty liver, which can then develop into alcoholic hepatitis, hepatic fibrosis and hepatic cirrhosis. Its main clinical features include nausea, vomiting, jaundice, and sometimes hepatomegaly and tenderness. Severe alcoholism can induce extensive hepatocyte necrosis or even hepatic failure. Alcoholic hepatopathy is one of common hepatic diseases in China, and seriously endangers people's health [1].

In addition to the toxic hepatic injury caused by alcohol, other "hepatotropic poisons" such as chemical toxicants and certain drugs in the environment can also cause hepatic injury. As an important detoxification organ for the human body, the liver has dual blood supply from the hepatic arteries and hepatic veins. Chemical substances can enter the liver through the portal veins of the gastrointestinal tract or the systemic circulation for conversion, and thus the liver is vulnerable to toxic substances in chemicals. There are substances that are toxic to the liver in both nature and human industrial production processes, known as "hepatotropic poisons". People are generally susceptible to these poisons, and they have a short incubation period. The pathological process is directly related to the amount of chemical substances, and these substances may cause different levels of hepatocyte necrosis, steatosis and hepatic cirrhosis in the liver. The pathological manifestations include (1) steatosis. Carbon tetrachloride, yellow phosphorus and the like can interfere with the synthesis and translocation of lipoproteins, forming fatty liver. (2) Lipid peroxidation, which is a special manifestation form of toxic hepatic injury. For example, carbon tetrachloride is metabolized *in vivo* to produce an intermediate product with strong oxidizing ability, leading to lipid peroxidation on biofilms, destructing membrane phospholipids and changing the structures and functions of cells. (3) Cholestasis, which is mainly associated with damage of the hepatic cell membrane and microvilli, causing bile acid excretion disorder [2].

Radiation can also cause hepatic tissue injury. In general, radiation sources are high-energy electromagnetic waves or high-energy particles produced by natural or artificial energy sources. Either instant irradiation with high-dose rays or prolonged irradiation with low-dose rays may cause tissue injury. The radiation energy destroys the chromosomes and enzymes of cells and disrupts the normal functions of cells.

Diabetic hepatic tissue injury refers to the lesions of liver histology and functions caused by diabetes mellitus. Hepatic injuries known to be caused by diabetes mellitus include: hepatic enzymology abnormalities, which can cause carbon dioxide accumulation, acidosis, reduced oxygen supply and increased oxygen consumption in hepatocytes, resulting in increased activity of liver transaminases and bilirubin metabolism disorder, with severe cases causing hepatocyte necrosis; fatty liver, wherein diabetes mellitus is the third most common cause of fatty liver among all causes of fatty liver, and 21%-78% of diabetics have fatty liver; and hepatitis, cirrhosis and hepatic carcinoma, wherein the prevalence of viral hepatitis in diabetics is about 2-4 times that in normal people, and the incidence of primary hepatic carcinoma is about 4 times that in normal people. Diabetic hepatopathy not only damages the quality of life of millions of patients, but also creates care needed for a huge burden cost and healthcare system strength.

Viral infection of the liver is also a common cause of hepatic injury, such as viral hepatitis B, viral hepatitis C, and viral hepatitis E.

Intrahepatic blood congestion may also cause hepatic tissue injury. Intrahepatic blood congestion is mainly caused by the following factors: hepatic veno-occlusive disease, Budd-Chiari syndrome, chronic right cardiac insufficiency and constrictive pericarditis.

Any disease that blocks the return of inferior vena cava blood to the heart can lead to liver congestion, such as rheumatic valvular heart disease, chronic constrictive pericarditis, hypertensive heart disease, ischemic heart disease, pulmonary heart disease and congenital heart disease.

Congestive hepatic injury initially affects the central lobule region; there are venous congestion and expansion in the central lobules, and the degree of hepatic sinusoid expansion varies depending on the distance between the hepatic sinusoids and veins in the central lobules; hepatocytes in the central lobules are compressed, deformed and atrophied; there are granular deformation in the cytoplasm, with nuclear condensation, nuclear division and cell necrosis, accompanied by brown pigmentation with the brown pigment located in the central lobules potentially caused by cholestasis; and degeneration and necrosis of the hepatic parenchyma near the central veins is the most serious, necrotic tissues extend toward the portal area with the exacerbation of congestion, patients with severe congestion only have normal liver tissues in the portal area, the reticular fibers around the central veins can collapse over time, and it can be seen that reticular fibrous tissues and fine fiber bundles extend from one central vein to another.

Currently, the treatment of hepatic tissue injury mainly includes the control and treatment of causes, as well as supportive treatment. Scientists have been looking for drugs that have a direct, good repair effect on injured liver tissues for a long time. The present inventors have also conducted intensive research on this. It is found through experiments that plasminogen, a protein substance naturally present in the human body, has a good repair effect on hepatic tissue injuries caused by poisoning, radiation, chemotherapeutic drugs and diabetes mellitus. Plasminogen is expected to become a new strategy for treating hepatic tissue injury and its related disorders.

Plasminogen (plg) is an inactive precursor of plasmin, a single-stranded glycoprotein that is composed of 791 amino acids and has a molecular weight of about 92 kDa [3,4]. Plasminogen is mainly synthesized in the liver and is abundantly present in the extracellular fluid. The content of plasminogen in plasma is about 2 µM. Therefore, plasminogen is a huge potential source of proteolytic activity in tissues and body fluids [5,6]. Plasminogen exists in two molecular forms: glutamic acid-plasminogen (Glu-plasminogen) and lysine-plasminogen (Lys-plasminogen). The naturally secreted and uncleaved forms of plasminogen have an amino-terminal (N-terminal) glutamic acid and are therefore referred to as glutamic acid-plasminogen. However, in the presence of plasmin, glutamic acid-plasminogen is hydrolyzed to lysine-plasminogen at Lys76-Lys77. Compared with glutamic acid-plasminogen, lysine-plasminogen has a higher affinity for fibrin and can be activated by PAs at a higher rate. The Arg560-Val561 peptide bond between these two forms of plasminogen can be cleaved by uPA or tPA, resulting in the formation of plasmin as a disulfide-linked double-strand protease [7]. The amino-terminal portion of plasminogen contains five homotrimeric rings, i.e., the so-called kringles, and the carboxy-terminal portion contains a protease domain. Some kringles contain lysine-binding sites that mediate the specific interaction of plasminogen with fibrin and its inhibitor α2-AP. A newly discovered 38 kDa fragment of plasminogen, comprising kringles 1-4, is a potent inhibitor of angiogenesis. This fragment is named as angiostatin and can be produced by the proteolysis of plasminogen via several proteases.

## Brief Description of the Invention

In one aspect, the present invention relates to a composition comprising an effective amount of plasminogen for use in the prevention and/or treatment of hepatic tissue injury caused by diabetes, radiation or chemical toxicants. In one embodiment, the hepatic tissue injury is hepatic injury caused by radiation or chemical substances. In one embodiment, the radiation or chemical substances causing hepatic injury are radiotherapy or chemotherapy methods and drugs used for the treatment of cancer. In one embodiment, the radiation is radiation caused by an accident or other event such as a work environment. In one embodiment, the hepatic tissue injury is toxic hepatic injury. In one embodiment, the toxic hepatic injury is toxic hepatic injury caused by "hepatotropic poisons" including alcohol. In one embodiment, the hepatic tissue injury is caused by diabetes mellitus and one of the complications of diabetes mellitus. In one embodiment, the hepatic tissue injury is drug-induced hepatic injury. In one embodiment, the hepatic tissue injury is diabetic hepatic injury, toxic hepatic injury, drug-induced hepatic injury, radiation-induced hepatic injuryIn one embodiment, the hepatic tissue injury includes hepatic dysfunction, abnormal hepatic enzymology, liver discomfort and haphalgesia, hepatomegaly, splenomegaly, hepatosplenomegaly, hepatitis, fatty liver, cholangitis, hepatic cirrhosis, hepatic necrosis and hepatic carcinoma caused by hepatic tissue injury.

In one embodiment, the sequence in this patent application refers to the patent document CN 102154253 A. In one embodiment, the plasminogen is a protein that comprises a plasminogen active fragment and still has plasminogen activity.

In one embodiment, the plasminogen is selected from Glu-plasminogen, Lys-plasminogen, mini-plasminogen, micro-plasminogen, δ-plasminogen or any combination thereof. In one embodiment, the plasminogen is for administration systemically or locally, including topical, intravenous, intramuscular, subcutaneous, inhalation, intraspinal, local injection, intraarticular injection or rectal administration. In one embodiment, the hepatic injury is caused by diabetes mellitus-induced angiopathy of large vessels, small vessels, and microvessels. In one embodiment, the plasminogen can be administered in combination with one or more other drugs. In one embodiment, the other drugs include: liver-protecting drugs, antidiabetic drugs, antithrombotic drugs, anticoagulant drugs, hypolipidemic drugs, drugs against cardiovascular and cerebrovascular diseases, and anti-infective drugs.

In one embodiment, the subject is a mammal, preferably human.

In one embodiment, the hepatic injury caused by diabetes mellitus is caused by diabetes mellitus-induced angiopathy of large vessels, small vessels, and microvessels.

In one embodiment, the subject has a low level of plasmin or plasminogen. Specifically, the low level is innate, secondary and/or local.

In one embodiment, the sequence in this patent application refers to the patent document CN 102154253 A. In one embodiment, the plasminogen is a protein that has 1-100, 1-90, 1-80, 1-70, 1-60, 1-50, 1-45, 1-40, 1-35, 1-30, 1-25, 1-20, 1-15, 1-10, 1-5, 1-4, 1-3, 1-2 or 1 amino acid added, deleted and/or substituted in SEQ ID No.2, 6, 8, 10 or 12, and still has the activity of plasminogen. In one embodiment, the plasminogen is a protein that comprises a plasminogen active fragment and still has the activity of plasminogen. In one embodiment, the plasminogen is selected from Glu-plasminogen, Lys-plasminogen, mini-plasminogen, micro-plasminogen, $\delta$-plasminogen or any combination thereof. In one embodiment, the plasminogen is a conservatively substituted variant selected from variants of Glu-plasminogen, Lys-plasminogen, mini-plasminogen, $\delta$-plasminogen or micro-plasminogen. In one embodiment, the plasminogen is a human natural plasminogen, such as an ortholog of plasminogen shown in SEQ ID No.2, e.g., an ortholog of plasminogen from primates or rodents, for example, an ortholog of plasminogen from gorillas, rhesus monkeys, murine, cows, horses and dogs. Most preferably, the amino acid sequence of the plasminogen of the present invention is as shown in SEQ ID No.2, 6, 8, 10 or 12.

In one embodiment, the plasminogen is for administration in combination with a suitable polypeptide carrier or stabilizer. In one embodiment, the plasminogen is administered at a dosage of 0.0001-2000 mg/kg, 0.001-800 mg/kg, 0.01-600 mg/kg, 0.1-400 mg/kg, 1-200 mg/kg, 1-100 mg/kg or 10-100 mg/kg (by per kg of body weight) or 0.0001-2000 mg/cm$^2$, 0.001-800 mg/cm$^2$, 0.01-600 mg/cm$^2$, 0.1-400 mg/cm$^2$, 1-200 mg/cm$^2$, 1-100 mg/cm$^2$ or 10-100 mg/cm$^2$ (by per square centimeter of body surface area) daily, preferably the dosage is repeated at least once, preferably the dosage is for administration at least daily. In the case of local administration, the above dosages may also be further adjusted depending on the circumstances.

In one embodiment, the plasminogen is for administration by systemic or topical route, preferably by the following routes: topical, intravenous, intramuscular, subcutaneous, inhalation, intraspinal, local injection, intraarticular injection or rectal route. In one embodiment, the local administration is performed by applying a plasminogen-containing catheter in the liver area.

In one aspect, the present invention relates to although does not claim a method for preventing and/or treating hepatic tissue injury and its related disorders in a subject, comprising administering an effective amount of plasminogen to the subject. The present invention also relates to the use of plasminogen for preventing and/or treating hepatic tissue injury in a subject.

In one embodiment, the hepatic tissue injury is hepatic injury caused by radiation or chemical substances. In one embodiment, the radiation or chemical substances causing hepatic injury is radiotherapy or chemotherapy methods and drugs used for the treatment of cancer. In one embodiment, the radiation is radiation caused by an accident event. In one embodiment, the hepatic tissue injury is toxic hepatic injury. In one embodiment, the toxic hepatic injury is toxic hepatic injury caused by "hepatotropic poisons" including alcohol. In one embodiment, the hepatic tissue injury is caused by diabetes mellitus and one of the complications of diabetes mellitus. In one embodiment, the hepatic tissue injury is due to hepatitis caused by viral infection of the liver, such as hepatitis caused by hepatitis A virus, hepatitis B virus, hepatitis C virus, hepatitis D virus or hepatitis E virus. In one embodiment, the hepatic tissue injury is drug-induced hepatic injury.. In one embodiment, the hepatic tissue injury is diabetic hepatic injury, toxic hepatic injury, drug-induced hepatic injury, or radiation-induced hepatic injury. In one embodiment, the hepatic tissue injury includes hepatic dysfunction, abnormal hepatic enzymology, liver discomfort and haphalgesia, hepatomegaly, splenomegaly, hepatosplenomegaly, hepatitis, fatty liver, cholangitis, hepatic cirrhosis, hepatic necrosis and hepatic carcinoma caused by hepatic tissue injury.

In one embodiment, the sequence in this patent application refers to the patent document CN 102154253 A. In one embodiment, the plasminogen is a protein that comprises a plasminogen active fragment and still has plasminogen activity. In one embodiment, the plasminogen is selected from Glu-plasminogen, Lys-plasminogen, mini-plasminogen, micro-plasminogen, $\delta$-plasminogen or any combination thereof. In one embodiment, the plasminogen is for administration systemically or locally, including topical, intravenous, intramuscular, subcutaneous, inhalation, intraspinal, local injection, intraarticular injection or rectal administration. In one embodiment, the hepatic injury is caused by diabetes mellitus-induced angiopathy of large vessels, small vessels, and microvessels. In one embodiment, the plasminogen is for administration in combination with one or more other drugs. In one embodiment, the other drugs include: liver-protecting drugs, antidiabetic drugs, antithrombotic drugs, anticoagulant drugs, hypolipidemic drugs, drugs against cardiovascular and cerebrovascular diseases, and anti-infective drugs.

In one embodiment, the subject is a mammal, preferably human.

In one embodiment, the hepatic injury caused by diabetes mellitus is caused by diabetes mellitus-induced angiopathy of large vessels, small vessels, and microvessels.

In one embodiment, the subject has a low level of plasmin or plasminogen. Specifically, the low level is innate, secondary and/or local.

In one embodiment, the sequence in this patent application refers to the patent document CN 102154253 A. In one embodiment, the plasminogen is a protein that has 1-100, 1-90, 1-80, 1-70, 1-60, 1-50, 1-45, 1-40, 1-35, 1-30, 1-25, 1-20, 1-15, 1-10, 1-5, 1-4, 1-3, 1-2 or 1 amino acid added, deleted and/or substituted in SEQ ID No.2, 6, 8, 10 or 12, and still has the activity of plasminogen. In one embodiment, the plasminogen is a protein that comprises a plasminogen active fragment and still has the activity of plasminogen. In one embodiment, the plasminogen is selected from Glu-plasminogen, Lys-plasminogen, mini-plasminogen, micro-plasminogen, δ-plasminogen or any combination thereof. In one embodiment, the plasminogen is a conservatively substituted variant selected from variants of Glu-plasminogen, Lys-plasminogen, mini-plasminogen, δ-plasminogen or micro-plasminogen. In one embodiment, the plasminogen is a human natural plasminogen, such as an ortholog of plasminogen shown in SEQ ID No.2, e.g., an ortholog of plasminogen from primates or rodents, for example, an ortholog of plasminogen from gorillas, rhesus monkeys, murine, cows, horses and dogs. Most preferably, the amino acid sequence of the plasminogen of the present invention is as shown in SEQ ID No.2, 6, 8, 10 or 12.

In one embodiment, the plasminogen is for administration in combination with a suitable polypeptide carrier or stabilizer. In one embodiment, the plasminogen is administered at a dosage of 0.0001-2000 mg/kg, 0.001-800 mg/kg, 0.01-600 mg/kg, 0.1-400 mg/kg, 1-200 mg/kg, 1-100 mg/kg or 10-100 mg/kg (by per kg of body weight) or 0.0001-2000 mg/cm$^2$, 0.001-800 mg/cm$^2$, 0.01-600 mg/cm$^2$, 0.1-400 mg/cm$^2$, 1-200 mg/cm$^2$, 1-100 mg/cm$^2$ or 10-100 mg/cm$^2$ (by per square centimeter of body surface area) daily, preferably the dosage is repeated at least once, preferably the dosage is administered at least daily. In the case of local administration, the above dosages may also be further adjusted depending on the circumstances.

In one embodiment, the plasminogen is for administration by systemic or topical route, preferably by the following routes: topical, intravenous, intramuscular, subcutaneous, inhalation, intraspinal, local injection, intraarticular injection or rectal route. In one embodiment, the local administration is performed by applying a plasminogen-containing catheter in the liver area.

The present invention relates to although does not claim plasminogen, a pharmaceutical composition comprising the plasminogen, or an article or kit comprising the plasminogen, which are useful in the prevention and/or treatment of hepatic tissue injury in a subject.

In one embodiment, the hepatic tissue injury is hepatic injury caused by radiation or chemical substances. In one embodiment, the radiation or chemical substances causing hepatic injury is radiotherapy or chemotherapy methods and drugs used for the treatment of cancer. In one embodiment, the radiation is radiation caused by an accident event. In one embodiment, the hepatic tissue injury is toxic hepatic injury. In one embodiment, the toxic hepatic injury is toxic hepatic injury caused by "hepatotropic poisons" including alcohol. In one embodiment, the hepatic tissue injury is caused by diabetes mellitus and one of the complications of diabetes mellitus. In one embodiment, the hepatic tissue injury is drug-induced hepatic injury. In one embodiment, the hepatic tissue injury is diabetic hepatic injury, toxic hepatic injury, drug-induced hepatic injury, or radiation-induced hepatic injury. In one embodiment, the hepatic tissue injury includes hepatic dysfunction, abnormal hepatic enzymology, liver discomfort and haphalgesia, hepatomegaly, splenomegaly, hepatosplenomegaly, hepatitis, fatty liver, cholangitis, hepatic cirrhosis, hepatic necrosis and hepatic carcinoma caused by hepatic tissue injury.

In one embodiment, the sequence in this patent application refers to the patent document CN 102154253 A. In one embodiment, the plasminogen is a protein that comprises a plasminogen active fragment and still has plasminogen activity. In one embodiment, the plasminogen is selected from Glu-plasminogen, Lys-plasminogen, mini-plasminogen, micro-plasminogen, δ-plasminogen or any combination thereof. In one embodiment, the plasminogen is for administration systemically or locally, including topical, intravenous, intramuscular, subcutaneous, inhalation, intraspinal, local injection, intraarticular injection or rectal administration. In one embodiment, the hepatic injury is caused by diabetes mellitus-induced angiopathy of large vessels, small vessels, and microvessels. In one embodiment, the plasminogen is for administration in combination with one or more other drugs. In one embodiment, the other drugs include: liver-protecting drugs, antidiabetic drugs, antithrombotic drugs, anticoagulant drugs, hypolipidemic drugs, drugs against cardiovascular and cerebrovascular diseases, and anti-infective drugs.

In one embodiment, the subject is a mammal, preferably human.

In one embodiment, the hepatic injury caused by diabetes mellitus is caused by diabetes mellitus-induced angiopathy of large vessels, small vessels, and microvessels.

In one embodiment, the subject has a low level of plasmin or plasminogen. Specifically, the low level is innate, secondary and/or local.

In one embodiment, the sequence in this patent application refers to the patent document CN 102154253 A. In one embodiment, the plasminogen is a protein that has 1-100, 1-90, 1-80, 1-70, 1-60, 1-50, 1-45, 1-40, 1-35, 1-30, 1-25, 1-20, 1-15, 1-10, 1-5, 1-4, 1-3, 1-2 or 1 amino acid added, deleted and/or substituted in SEQ ID No.2, 6, 8, 10 or 12, and still has the activity of plasminogen. In one embodiment, the plasminogen is a protein that comprises a plasminogen active fragment and still has the activity of plasminogen. In one embodiment, the plasminogen is selected from Glu-plasminogen, Lys-plasminogen, mini-plasminogen, micro-plasminogen, δ-plasminogen or any combination thereof. In one embodiment, the plasminogen is a conservatively substituted variant selected from variants of Glu-plasminogen, Lys-plasminogen, mini-plasminogen, δ-plasminogen or micro-plasminogen. In one embodiment, the plasminogen is a human natural plasminogen, such as an ortholog of plasminogen shown in SEQ ID No.2, e.g., an ortholog of plasminogen from primates or rodents, for example, an ortholog of plasminogen from gorillas, rhesus monkeys, murine, cows, horses and dogs. Most preferably, the amino acid sequence of the plasminogen of the present invention is as shown in SEQ ID No.2, 6, 8, 10 or 12.

In one embodiment, the plasminogen is for administration in combination with a suitable polypeptide carrier or stabilizer. In one embodiment, the plasminogen is for administration at a dosage of 0.0001-2000 mg/kg, 0.001-800 mg/kg, 0.01-600 mg/kg, 0.1-400 mg/kg, 1-200 mg/kg, 1-100 mg/kg or 10-100 mg/kg (by per kg of body weight) or 0.0001-2000 mg/cm$^2$, 0.001-800 mg/cm$^2$, 0.01-600 mg/cm$^2$, 0.1-400 mg/cm$^2$, 1-200 mg/cm$^2$, 1-100 mg/cm$^2$ or 10-100 mg/cm$^2$ (by per square centimeter of body surface area) daily, preferably the dosage is repeated at least once, preferably the dosage is for administration at least daily. In the case of local administration, the above dosages may also be further adjusted depending on the circumstances.

In one embodiment, the plasminogen is for administration by systemic or topical route, preferably by the following routes: topical, intravenous, intramuscular, subcutaneous, inhalation, intraspinal, local injection, intraarticular injection or rectal route. In one embodiment, the local administration is performed by applying a plasminogen-containing catheter in the liver area.

In one embodiment, the plasminogen is subpackaged in containers. Preferably, the article or kit further comprises other drugs subpackaged in other containers of the kit. The kit can also comprise instructions for use, which indicate that the plasminogen can be used to treat hepatic tissue injury, specifically, for example, diabetic hepatic injury caused by diabetes mellitus, toxic hepatic injury, drug-induced hepatic injury, hepatic injury caused by radiation, or hepatic injury and can further indicate that the plasminogen can be administered before, simultaneously with and/or after administration of other drugs or therapies.

## Detailed Description of Embodiments

"Diabetic hepatic injury" refers to a lesion with the histological and functional changes of the liver caused by diabetes mellitus. It is mainly caused by diabetes mellitus-induced angiopathy of large vessels, small vessels, and microvessels. Hepatic injuries known to be caused by diabetes mellitus include: hepatic enzymology abnormalities, which can cause carbon dioxide accumulation, acidosis, reduced oxygen supply and increased oxygen consumption in hepatocytes, resulting in increased activity of liver transaminases and bilirubin metabolism disorder, with severe cases causing hepatocyte necrosis; fatty liver, wherein diabetes mellitus is the third most common cause of fatty liver among all causes of fatty liver, and 21%-78% of diabetics have fatty liver; and hepatitis, cirrhosis and hepatic carcinoma, wherein the prevalence of viral hepatitis in diabetics is about 2-4 times that in normal people, and the incidence of primary hepatic carcinoma is about 4 times that in normal people.

"Chemical hepatic injury" or "toxic hepatic injury" refers to hepatic damage caused by chemical hepatotoxic substances. These chemical substances include alcohol, chemical toxicants in the environment and certain drugs. There are substances that are toxic to the liver in both nature and human industrial production processes, known as "hepatotropic poisons". People are generally susceptible to these poisons, and they have a short incubation period. The pathological process is directly related to the amount of chemical substances, and these substances may cause different levels of hepatocyte necrosis, steatosis and hepatic cirrhosis in the liver.

"hepatotropic poisons" refer to a generic term for substances that are toxic to the liver. Alcohol is the most common "hepatotropic poison" in life. In addition to alcohol, chemical toxicants in the environment and certain drugs may also cause hepatic injury. As an important detoxification organ for the human body, the liver has dual blood supply from the hepatic arteries and hepatic veins. Chemical substances can enter the liver through the portal veins of the gastrointestinal tract or the systemic circulation for conversion, and thus the liver is vulnerable to toxic substances in chemicals. There are substances that are toxic to the liver in both nature and human industrial production processes, known as "hepatotropic poisons". They enter the liver and may cause different levels of hepatocyte necrosis, steatosis and hepatic cirrhosis in the liver. The pathological manifestations include (1) steatosis. Carbon tetrachloride, yellow phosphorus and the like can interfere with the synthesis and translocation of lipoproteins, forming fatty liver. (2) Lipid peroxidation, which is a special manifestation form of toxic hepatic injury. For example, carbon tetrachloride is metabolized *in vivo* to produce an intermediate product with strong oxidizing ability, leading to lipid peroxidation on biofilms, destructing membrane phospholipids and changing the structures and functions of cells. (3) Cholestasis, which is mainly associated with damage of the hepatic cell membrane and microvilli, causing bile acid excretion disorder.

"Drug-induced hepatic injury", also known as drug-induced hepatopathy, refers to hepatic injury during drug use caused by the drug itself or/and its metabolites, or the hypersensitivity or reduced tolerance to the drug due to a special constitution of the body, clinically manifested as a variety of acute and chronic hepatopathies. Patients with light illness may recover spontaneously after discontinuation of medication, and serious ones may be life-threatening and require active treatment and rescue. "Drug-induced hepatic injury" can occur in a healthy person who has not previously had a history of hepatopathy or a patient who has had a serious disease previously. It can occur when the medication is overdosed, but it can also occur under normal dosages.

"Radiation-induced hepatic injury" refers to radiation injury caused by high-energy ionizing radiation, including $\alpha$ and $\beta$ particles, $\gamma$ rays, $\chi$ rays and neutron rays. Either instant irradiation with high-dose rays or prolonged irradiation with low-dose rays may cause tissue injury. The radiation energy destroys the chromosomes and enzymes of cells and disrupts the normal functions of cells.

"Plasminogen" is the zymogenic form of plasmin, and based on the sequence in the swiss prot and calculated from the amino acid sequence (SEQ ID No.4) of the natural human-derived plasminogen containing a signal peptide, is a glycoprotein composed of 810 amino acids, which has a molecular weight of about 92 kD and is synthesized mainly in the liver and capable of circulating in the blood; and the cDNA sequence encoding this amino acid sequence is as shown in SEQ ID No3. Full-length plasminogen contains seven domains: a C-terminal serine protease domain, an N-terminal Pan Apple (PAp) domain and five Kringle domains (Kringles 1-5). Referring to the sequence in the swiss prot, the signal peptide comprises residues Met1-Gly19, PAp comprises residues Glu20-Val98, Kringle 1 comprises residues Cys103-Cys181, Kringle 2 comprises residues Glu184-Cys262, Kringle 3 comprises residues Cys275-Cys352, Kringle 4 comprises residues Cys377-Cys454, and Kringle 5 comprises residues Cys481-Cys560. According to the NCBI data, the serine protease domain comprises residues Val581-Arg804.

Glu-plasminogen is a natural full-length plasminogen and is composed of 791 amino acids (without a signal peptide of 19 amino acids); the cDNA sequence encoding this sequence is as shown in sequence 1; and the amino acid sequence is as shown in SEQ ID No.2. In vivo, Lys-plasminogen, which is formed by hydrolysis of amino acids at positions 76-77 of Glu-plasminogen, is also present, as shown in SEQ ID No.6; and the cDNA sequence encoding this amino acid sequence is as shown in SEQ ID No.5. δ-plasminogen is a fragment of full-length plasminogen that lacks the structure of Kringle 2-Kringle 5 and contains only Kringle 1 and the serine protease domain [8,9]. The amino acid sequence (SEQ ID No.8) of δ-plasminogen has been reported in the literature [9], and the cDNA sequence encoding this amino acid sequence is as shown in SEQ ID No.7. Mini-plasminogen is composed of Kringle 5 and the serine protease domain, and has been reported in the literature to comprise residues Val443-Asn791 (with the Glu residue of the Glu-plasminogen sequence that does not contain a signal peptide as the starting amino acid) [10]; the amino acid sequence is as shown in SEQ ID No. 10; and the cDNA sequence encoding this amino acid sequence is as shown in SEQ ID No.9. Micro-plasminogen comprises only the serine protease domain, the amino acid sequence of which has been reported in the literature to comprise residues Ala543-Asn791 (with the Glu residue of the Glu-plasminogen sequence that does not contain a signal peptide as the starting amino acid) [11], and the sequence of which has been also reported in patent document CN 102154253 A to comprise residues Lys531-Asn791 (with the Glu residue of the Glu-plasminogen sequence that does not contain a signal peptide as the starting amino acid) (the sequence in this patent application refers to the patent document CN 102154253 A); the amino acid sequence is as shown in SEQ ID No.12; and the cDNA sequence encoding this amino acid sequence is as shown in SEQ ID No.11.

In the present invention, "plasmin" is used interchangeably with "fibrinolysin" and "fibrinoclase", and the terms have the same meaning; and "plasminogen" is used interchangeably with "fibrinolytic zymogen" and "fibrinoclase zymogen", and the terms have the same meaning.

Those skilled in the art can understand that all the technical solutions of the plasminogen of the present invention are suitable for plasmin. Therefore, the technical solutions described in the present invention cover plasminogen and plasmin.

In the course of circulation, plasminogen is in a closed, inactive conformation, but when bound to thrombi or cell surfaces, it is converted into an active plasmin in an open conformation under the mediation of a plasminogen activator (PA). The active plasmin can further hydrolyze the fibrin clots to fibrin degradation products and D-dimers, thereby dissolving the thrombi. The PAp domain of plasminogen comprises an important determinant that maintains plasminogen in an inactive, closed conformation, and the KR domain is capable of binding to lysine residues present on receptors and substrates. A variety of enzymes that can serve as plasminogen activators are known, including: tissue plasminogen activator (tPA), urokinase plasminogen activator (uPA), kallikrein, coagulation factor XII (Hagmann factor), and the like.

"Plasminogen active fragment" refers to an active fragment in the plasminogen protein that is capable of binding to a target sequence in a substrate and exerting the proteolytic function. The technical solutions of the present invention involving plasminogen encompass technical solutions in which plasminogen is replaced with a plasminogen active fragment. The plasminogen active fragment of the present invention is a protein comprising a serine protease domain of plasminogen. Preferably, the plasminogen active fragment of the present invention comprises SEQ ID NO: 14, or an amino acid sequence having an amino acid sequence identity of at least 80%, 90%, 95%, 96%, 97%, 98% or 99% with SEQ ID NO: 14. Therefore, plasminogen of the present invention comprises a protein comprising the plasminogen active fragment and still having plasminogen activity.

At present, methods for determining plasminogen and its activity in blood include: detection of tissue plasminogen activator activity (t-PAA), detection of tissue plasminogen activator antigen (t-PAAg) in plasma, detection of tissue plasminogen activity (plgA) in plasma, detection of tissue plasminogen antigen (plgAg) in plasma, detection of activity of the inhibitor of tissue plasminogen activators in plasma, detection of inhibitor antigens of tissue plasminogen activators in plasma and detection of plasmin-anti-plasmin (PAP) complex in plasma. The most commonly used detection method is the chromogenic substrate method: streptokinase (SK) and a chromogenic substrate are added to a test plasma, the PLG in the test plasma is converted into PLM by the action of SK, PLM acts on the chromogenic substrate, and then it is determined that the increase in absorbance is directly proportional to plasminogen activity using a spectrophotometer. In addition, plasminogen activity in blood can also be determined by immunochemistry, gel electrophoresis, immunonephelometry, radioimmuno-diffusion and the like.

"Orthologues or orthologs" refer to homologs between different species, including both protein homologs and DNA homologs, and are also known as orthologous homologs and vertical homologs. The term specifically refers to proteins or genes that have evolved from the same ancestral gene in different species. The plasminogen of the present invention includes human natural plasminogen, and also includes orthologues or orthologs of plasminogens derived from different species and having plasminogen activity.

"Conservatively substituted variant" refers to one in which a given amino acid residue is changed without altering the overall conformation and function of the protein or enzyme, including, but not limited to, replacing an amino acid in the amino acid sequence of the parent protein by an amino acid with similar properties (such as acidity, alkalinity and hydrophobicity). Amino acids with similar properties are well known. For example, arginine, histidine and lysine are hydrophilic basic amino acids and are interchangeable. Similarly, isoleucine is a hydrophobic amino acid that can be replaced by leucine, methionine or valine. Therefore, the similarity of two proteins or amino acid sequences with similar functions may be different. For example, the similarity (identity) is 70%-99% based on the MEGALIGN algorithm. "Conservatively substituted variant" also includes a polypeptide or enzyme having amino acid identity of 60% or more, preferably 75% or more, more preferably 85% or more, even more preferably 90% or more as determined by the BLAST or FASTA algorithm, and having the same or substantially similar properties or functions as the natural or parent protein or enzyme.

"Isolated" plasminogen refers to the plasminogen protein that is isolated and/or recovered from its natural environment. In some embodiments, the plasminogen will be purified (1) to a purity of greater than 90%, greater than 95% or greater than 98% (by weight), as determined by the Lowry method, such as more than 99% (by weight); (2) to a degree sufficiently to obtain at least 15 residues of the N-terminal or internal amino acid sequence using a spinning cup sequenator; or (3) to homogeneity, which is determined by sodium dodecyl sulfate-polyacrylamide gel electrophoresis (SDS-PAGE) under reducing or nonreducing conditions using Coomassie blue or silver staining. Isolated plasminogen also includes plasminogen prepared from recombinant cells by bioengineering techniques and separated by at least one purification step.

The terms "polypeptide", "peptide" and "protein" are used interchangeably herein and refer to polymeric forms of amino acids of any length, which may include genetically encoded and non-genetically encoded amino acids, chemically or biochemically modified or derivatized amino acids, and polypeptides having modified peptide backbones. The term includes fusion proteins, including, but not limited to, fusion proteins having heterologous amino acid sequences, fusions having heterologous and homologous leader sequences (with or without N-terminal methionine residues), and the like.

The "percent amino acid sequence identity (%)" with respect to the reference polypeptide sequence is defined as the percentage of amino acid residues in the candidate sequence identical to the amino acid residues in the reference polypeptide sequence when a gap is introduced as necessary to achieve maximal percent sequence identity and no conservative substitutions are considered as part of sequence identity. The comparison for purposes of determining percent amino acid sequence identity can be achieved in a variety of ways within the skill in the art, for example using publicly available computer softwares, such as BLAST, BLAST-2, ALIGN or Megalign (DNASTAR) software. Those skilled in the art can determine appropriate parameters for aligning sequences, including any algorithm needed to achieve the maximum comparison over the full length of the sequences being compared. However, for purposes of the present invention, the percent amino acid sequence identity value is generated using the sequence comparison computer program ALIGN-2.

In the case of comparing amino acid sequences using ALIGN-2, the % amino acid sequence identity of a given amino acid sequence A relative to a given amino acid sequence B (or may be expressed as a given amino acid sequence A having or containing a certain % amino acid sequence identity relative to, with or for a given amino acid sequence B) is calculated as follows:

$$\text{fraction } X/Y \times 100$$

wherein X is the number of identically matched amino acid residues scored by the sequence alignment program ALIGN-2 in the alignment of A and B using the program, and wherein Y is the total number of amino acid residues in B. It will be appreciated that where the length of amino acid sequence A is not equal to the length of amino acid sequence B, the % amino acid sequence identity of A relative to B will not be equal to the % amino acid sequence identity of B relative to A. Unless specifically stated otherwise, all the % amino acid sequence identity values used herein are obtained using the ALIGN-2 computer program as described in the previous paragraph.

As used herein, the terms "treatment" and "treating" refer to obtaining a desired pharmacological and/or physiologic effect. The effect may be complete or partial prevention of a disease or its symptoms and/or partial or complete cure of the disease and/or its symptoms, and includes: (a) prevention of the disease from developing in a subject that may have a predisposition to the disease but has not been diagnosed as having the disease; (b) suppression of the disease, i.e., blocking its formation; and (c) alleviation of the disease and/or its symptoms, i.e., eliminating the disease and/or its symptoms.

The terms "individual", "subject" and "patient" are used interchangeably herein and refer to mammals, including, but not limited to, murine (rats and mice), non-human primates, humans, dogs, cats , hoofed animals (e.g., horses, cattle, sheep, pigs, goats) and so on.

"Therapeutically effective amount" or "effective amount" refers to an amount of plasminogen sufficient to achieve the prevention and/or treatment of a disease when administered to a mammal or another subject to treat the disease. The "therapeutically effective amount" will vary depending on the plasminogen used, the severity of the disease and/or its symptoms, as well as the age, body weight of the subject to be treated, and the like.

**Preparation of the plasminogen of the present invention**

Plasminogen can be isolated and purified from nature for further therapeutic uses, and can also be synthesized by standard chemical peptide synthesis techniques. When chemically synthesized, a polypeptide can be subjected to liquid or solid phase synthesis. Solid phase polypeptide synthesis (SPPS) is a method suitable for chemical synthesis of plasminogen, in which the C-terminal amino acid of a sequence is attached to an insoluble support, followed by the sequential addition of the remaining amino acids in the sequence. Various forms of SPPS, such as Fmoc and Boc, can be used to synthesize plasminogen. Techniques for solid phase synthesis are described in Barany and Solid-Phase Peptide Synthesis; pp. 3-284 in The Peptides: Analysis, Synthesis, Biology. Vol. 2: Special Methods in Peptide Synthesis, Part A., Merrifield et al. J. Am. Chem. Soc., 85: 2149-2156 (1963); Stewart et al. Solid Phase Peptide Synthesis, 2nd ed. Pierce Chem. Co., Rockford, Ill. (1984); and Ganesan A. 2006 Mini Rev. Med Chem. 6:3-10 and Camarero JA et al. 2005 Protein Pept Lett. 12:723-8. Briefly, small insoluble porous beads are treated with a functional unit on which a peptide chain is constructed. After repeated cycles of coupling/deprotection, the attached solid phase free N-terminal amine is coupled to a single N-protected amino acid unit. This unit is then deprotected to expose a new N-terminal amine that can be attached to another amino acid. The peptide remains immobilized on the solid phase before it is cut off.

Standard recombinant methods can be used to produce the plasminogen of the present invention. For example, a nucleic acid encoding plasminogen is inserted into an expression vector, so that it is operably linked to a regulatory sequence in the expression vector. Expression regulatory sequence includes, but is not limited to, promoters (e.g., naturally associated or heterologous promoters), signal sequences, enhancer elements and transcription termination sequences. Expression regulation can be a eukaryotic promoter system in a vector that is capable of transforming or transfecting eukaryotic host cells (e.g., COS or CHO cells). Once the vector is incorporated into a suitable host, the host is maintained under conditions suitable for high-level expression of the nucleotide sequence and collection and purification of plasminogen.

A suitable expression vector is usually replicated in a host organism as an episome or as an integral part of the host chromosomal DNA. In general, an expression vector contains a selective marker (e.g., ampicillin resistance, hygromycin resistance, tetracycline resistance, kanamycin resistance or neomycin resistance) to facilitate detection of those exogenous cells transformed with a desired DNA sequence.

*Escherichia coli* is an example of prokaryotic host cells that can be used to clone a polynucleotide encoding the subject antibody. Other microbial hosts suitable for use include *Bacillus,* for example, *Bacillus subtilis* and other species of *Enterobacteriaceae* (such as *Salmonella* spp. and *Serratia* spp.), and various *Pseudomonas* spp. In these prokaryotic hosts, expression vectors can also be generated which will typically contain an expression control sequence (e.g., origin of replication) that is compatible with the host cell. In addition, there will be many well-known promoters, such as the lactose promoter system, the tryptophan (trp) promoter system, the beta-lactamase promoter system or the promoter system from phage lambda. Optionally in the case of manipulation of a gene sequence, a promoter will usually control expression, and has a ribosome binding site sequence and the like to initiate and complete transcription and translation.

Other microorganisms, such as yeast, can also be used for expression. *Saccharomyces* (e.g., *S. cere-*

*visiae*) and *Pichia* are examples of suitable yeast host cells, in which a suitable vector has an expression control sequence (e.g., promoter), an origin of replication, a termination sequence and the like, as required. A typical promoter comprises 3-phosphoglycerate kinase and other glycolytic enzymes. Inducible yeast promoters specifically include promoters derived from alcohol dehydrogenase, isocytochrome C, and enzymes responsible for maltose and galactose utilization.

In addition to microorganisms, mammalian cells (eg, mammalian cells cultured in in vitro cell culture) can also be used to express and produce the protein of the invention (eg, polynucleotides encoding the subject protein). See Winnacker, From Genes to Clones, VCH Publishers, N.Y., N.Y. (1987). Suitable mammalian host cells include CHO cell lines, various Cos cell lines, HeLa cells, myeloma cell lines and transformed B cells or hybridomas. Expression vectors for these cells may comprise an expression control sequence, such as an origin of replication, promoter and enhancer (Queen et al. Immunol. Rev. 89:49 (1986)), as well as necessary processing information sites, such as a ribosome binding site, RNA splice site, polyadenylation site and transcription terminator sequence. Examples of suitable expression control sequences are promoters derived from white immunoglobulin gene, SV40, adenovirus, bovine papilloma virus, cytomegalovirus and the like. See Co et al. J. Immunol. 148:1149 (1992).

Once synthesized (chemically or recombinantly), the plasminogen of the present invention can be purified according to standard procedures in the art, including ammonium sulfate precipitation, affinity column, column chromatography, high performance liquid chromatography (HPLC), gel electrophoresis and the like. The plasminogen is substantially pure, e.g., at least about 80% to 85% pure, at least about 85% to 90% pure, at least about 90% to 95% pure, or 98% to 99% pure or purer, for example free of contaminants such as cell debris, macromolecules other than the plasminogen and the like.

**Pharmaceutical formulations**

A therapeutic formulation can be prepared by mixing plasminogen of a desired purity with an optional pharmaceutical carrier, excipient or stabilizer (Remington's Pharmaceutical Sciences, 16th edition, Osol, A. ed. (1980)) to form a lyophilized preparation or an aqueous solution. Acceptable carriers, excipients and stabilizers are non-toxic to the recipient at the dosages and concentrations employed, and include buffers, such as phosphates, citrates and other organic acids; antioxidants, including ascorbic acid and methionine; preservatives (e.g., octadecyl dimethyl benzyl ammonium chloride; hexane chloride diamine; benzalkonium chloride and benzethonium chloride; phenol, butanol or benzyl alcohol; alkyl p-hydroxybenzoates, such as methyl or propyl p-hydroxybenzoate; catechol; resorcinol; cyclohexanol;

3-pentanol; and m-cresol); low molecular weight polypeptides (less than about 10 residues); proteins, such as serum albumin, gelatin or immunoglobulins; hydrophilic polymers, such as polyvinylpyrrolidone; amino acids, such as glycine, glutamine, asparagine, histidine, arginine or lysine; monosaccharides, disaccharides and other carbohydrates, including glucose, mannose or dextrins; chelating agents, such as EDTA; sugars, such as sucrose, mannitol, fucose or sorbitol; salt-forming counterions, such as sodium; metal complexes (e.g., zinc-protein complexes); and/or non-ionic surfactants, such as TWEENTM, PLURONICSTM or polyethylene glycol (PEG).

The formulations of the invention may also comprise one or more active compounds required for the particular disorder to be treated, preferably those that are complementary in activity and have no side effects with one another, for example anti-hypertensive drugs, anti-arrhythmic drugs, drugs for treating diabetes mellitus, and the like.

The plasminogen of the present invention may be encapsulated in microcapsules prepared by techniques such as coacervation or interfacial polymerization, for example, it may be incorporated in a colloid drug delivery system (e.g., liposomes, albumin microspheres, microemulsions, nanoparticles and nanocapsules), or incorporated in hydroxymethylcellulose or gel-microcapsules and poly-(methyl methacrylate) microcapsules in macroemulsions. These techniques are disclosed in Remington's Pharmaceutical Sciences, 16th edition, Osol, A. Ed. (1980).

The plasminogen of the present invention for *in vivo* administration must be sterile. This can be easily achieved by filtration through a sterile filtration membrane before or after freeze drying and reconstitution.

The plasminogen of the present invention can be prepared into a sustained-release preparation. Suitable examples of sustained-release preparations include solid hydrophobic polymer semi-permeable matrices having a shape and containing glycoproteins, such as films or microcapsules. Examples of sustained-release matrices include polyesters, hydrogels (e.g., poly(2-hydroxyethylmethacrylate)) (Langer et al. J. Biomed. Mater. Res., 15: 167-277 (1981); and Langer, Chem. Tech., 12:98-105 (1982)), or poly(vinyl alcohol), polylactides (US Patent 3773919, and EP 58, 481), copolymer of L-glutamic acid and γ ethyl-L-glutamic acid (Sidman et al. Biopolymers 22:547(1983)), nondegradable ethylene-vinyl acetate (Langer et al. *supra*), or degradable lactic acid-glycolic acid copolymers such as Lupron DepotTM (injectable microspheres composed of lactic acid-glycolic acid copolymer and leuprolide acetate), and poly D-(-)-3-hydroxybutyric acid. Polymers, such as ethylene-vinyl acetate and lactic acid-glycolic acid, are able to persistently release molecules for 100 days or longer, while some hydrogels release proteins for a shorter period of time. A rational strategy for protein stabilization can be designed based on relevant mechanisms. For example, if the aggregation mechanism is discovered to be formation of an intermolecular S-S bond through thio-disulfide interchange, stability is achieved by modifying sulfhydryl residues, lyophilizing from acidic solutions, controlling moisture content, using appropriate additives, and developing specific polymer matrix compositions.

## Administration and dosage

The pharmaceutical composition of the present invention can be administered in different ways, for example by intravenous, intraperitoneal, subcutaneous, intracranial, intrathecal, intraarterial (e.g., via carotid), intramuscular, intranasal, topical or intradermal administration or spinal cord or brain delivery. An aerosol preparation, such as a nasal spray preparation, comprises purified aqueous or other solutions of the active agent along with a preservative and isotonic agent. Such preparations are adjusted to a pH and isotonic state compatible with the nasal mucosa.

Preparations for parenteral administration include sterile aqueous or non-aqueous solutions, suspensions and emulsions. Examples of non-aqueous solvents are propylene glycol, polyethylene glycol, vegetable oils such as olive oil, and injectable organic esters such as ethyl oleate. Aqueous carriers include water, and alcoholic/aqueous solutions, emulsions or suspensions, including saline and buffered media. Parenteral vehicles include sodium chloride solution, Ringer's dextrose, dextrose and sodium chloride, or fixed oils. Intravenous vehicles include liquid and nutrient supplements, electrolyte supplements and the like. Preservatives and other additives may also be present, for example, such as antimicrobial agents, antioxidants, chelating agents and inert gases.

The medical staff will determine the dosage regimen based on various clinical factors. As is well known in the medical field, the dosage of any patient depends on a variety of factors, including the patient's size, body surface area, age, the specific compound to be administered, sex, frequency and route of administration, overall health and other drugs administered simultaneously. The dosage range of the pharmaceutical composition comprising plasminogen of the present invention may be, for example, about 0.0001 to 2000 mg/kg, or about 0.001 to 500 mg/kg (such as 0.02 mg/kg, 0.25 mg/kg, 0.5 mg/kg, 0.75 mg/kg, 10 mg/kg and 50 mg/kg) of the subject's body weight daily. For example, the dosage may be 1 mg/kg body weight or 50 mg/kg body weight, or in the range of 1 mg/kg-50 mg/kg, or at least 1 mg/kg. Dosages above or below this exemplary range are also contemplated, especially considering the above factors. The intermediate dosages in the above range are also included in the scope of the present invention. A subject may be administered with such dosages daily, every other day, weekly or based on any other schedule determined by empirical analysis. An exemplary dosage schedule includes 1-10 mg/kg for consecutive days. During administration of the drug of the present invention, the therapeutic effect and safety of diabetic hepatopathy and its related disorders are required to be assessed real-timely and regularly.

## Treatment efficacy and treatment safety

One embodiment of the present invention relates to the judgment of treatment efficacy and treatment safety after treating a subject with plasminogen. The methods for judging the treatment efficacy include, but are not limited to: 1) examining the liver function of a subject, for example, enzymatic levels in the patient, such as levels of serum aspartate aminotransferase (AST), alanine transaminase (ALT), total bilirubin, direct bilirubin, indirect bilirubin, albumin, globulin, cholinesterase, alkaline phosphatase and transpeptidase, are examined to determine whether they are in the normal value ranges or not, and after treating the subject with plasminogen of the present invention, it is expected that the above liver function indexes will return to normal value or be improved, such as alanine transaminase (ALT) at 0-40 $\mu$/L, aspartate transaminase (AST) at 0-40 $\mu$/L, gamma glutamyl transferase (GGT) of less than 40 units and total bilirubin at 3.4-20.5 $\mu$mol/L; 2) examining the prothrombin time (PT) and prothrombin activity (PTA) of the subject: PT is an important index reflecting the function of hepatic coagulation factor synthesis; PTA is a commonly used expression method for PT measurement value and of great value in judging the progression and prognosis of hepatopathy, in which the progressive decrease of PTA to less than 40% is an important diagnostic criterion for hepatic failure, and to less than 20% indicates hepatic dysfunction; and after treating the subject with plasminogen and its variants of the present invention, the decrease in PTA of the patient is expected to be remarkably improved; 3) imageological examination: including abdominal color ultrasonography of the liver, gallbladder and spleen, CT or MRI to learn the degree of recovery of hepatic injury; 4) examining tumor markers, such as alpha fetal protein (AFP), CA199 and AFU; and 5) hepatic biopsy to determine the degree of recovery of fibrosis and other injuries. In addition, the present invention also relates to the judgment of the safety of the therapeutic regimen during and after treating a subject with plasminogen and its variants, including, but not limited to, statistics of serum half-life, half-life of treatment, median toxic dose (TD50) and median lethal dose (LD50) in the subject, or observing various adverse events such as sensitization that occur during or after treatment This embodiment is not covered by the claimed invention.

## Articles or kits

One embodiment of the present invention relates to although does not claim an article or kit comprising plasminogen of the present invention useful in the treatment of the hepatic injury caused by diabetes mellitus. The article preferably includes a container, label or package insert. Suitable containers include bottles, vials, syringes and the like. The container can be made of various materials, such as glass or plastic. The container contains a composition that is effective to treat the disease or disorder of the present invention and has a sterile access (for example, the container may be an intravenous solution bag or vial containing a plug that can be pierced by a hypodermic injection needle). At least one active agent in the composition is plasminogen. The label on or attached to the container indicates that the composition is used to treat the hepatic injury caused by diabetes mellitus according to the present invention. The article may further comprise a second container containing a pharmaceutically acceptable buffer, such as phosphate buffered saline, Ringer's solution and glucose solution. It may further comprise other substances required from a commercial and user perspective, including other buffers, diluents, filters, needles and syringes. In addition, the article comprises a package insert with instructions for use, including, for example, instructions to a user of the composition to administer the plasminogen composition and other drugs to treat an accompanying disease to a patient. This embodiment is not covered by the claimed invention.

**Brief Description of the Drawings**

Figure 1 shows changes in body weight after administration of plasminogen to 24-25-week-old diabetic mice.

Figure 2 shows the observed results of HE staining of the liver after administration of plasminogen to 24-25-week-old diabetic mice for 15 consecutive days.

Figure 3 shows the observed results of fibrin immunostaining of the liver under a microscopy after administration of plasminogen to 24-25-week-old diabetic mice for 15 consecutive days.

Figure 4 shows changes in body weight after administration of plasminogen to 24-25-week-old diabetic mice for 31 consecutive days.

Figure 5 shows the observed results of HE staining of the liver after administration of plasminogen to 24-25-week-old diabetic mice for 31 consecutive days.

Figure 6 shows the observed results of fibrin immunostaining of the liver after administration of plasminogen to 24-25-week-old diabetic mice for 31 consecutive days.

Figure 7 shows the observed results of F4/80 immunostaining of the liver after administration of plasminogen to 24-25-week-old diabetic mice for 31 consecutive days.

Figure 8 shows the detection results of alanine transaminase (ALT) in serum after administration of PBS or plasminogen to 24-25-week-old diabetic mice for 31 days.

Figure 9 shows the observed results of HE staining of the liver on days 0, 2 and 7 after administration of plasminogen to mice with acute hepatic injury induced by carbon tetrachloride.

Figure 10 shows the observed results of HE staining of the liver at hours 18, 24 and 48 after administration of plasminogen to plg$^{-/-}$ mice with acute hepatic injury induced by carbon tetrachloride.

Figure 11 shows the observed results of fibrin immunostaining of the liver at hours 18, 24 and 48 after administration of plasminogen to plg$^{-/-}$ mice with acute hepatic injury induced by carbon tetrachloride.

Figure 12 shows the observed results of F4/80 immunostaining of the liver 10 days after administration of plasminogen to mice irradiated with 5.0 Gy X-rays.

Figure 13 shows the observed results of fibrin immunostaining of the liver 7 days after administration of plasminogen to model mice with injury induced by 10 mg/Kg cisplatin chemotherapy.

Figure 14 shows the observed results of HE staining of the liver at hours 18, 24 and 48 and 7 days after administration of plasminogen to plg$^{-/-}$ mice with acute hepatic injury induced by carbon tetrachloride.

**Examples**

**Example 1. Effect of plasminogen on body weight of late diabetic mice with nerve injury**

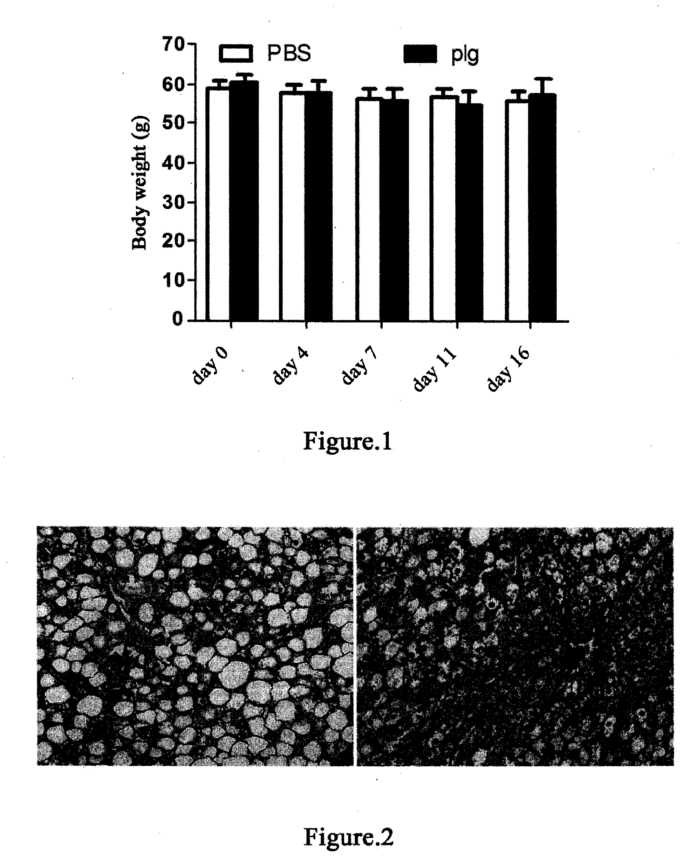

Ten male db/db mice aged 24-25 weeks were randomly divided into two groups, five in the control group administered with vehicle PBS and five in the group administered with plasminogen, respectively. The day when the experiment began was recorded on Day 0, and the mice were weighed and grouped. From the second day of the experiment, plasminogen or PBS was administered to the mice for 15 consecutive days, and the day was recorded as Day 1. Mice in the group administered with plasminogen were injected with plasminogen at a dose of 2 mg/0.2 mL/mouse/day via the tail vein, and an equal volume of PBS was administered to mice in the control group administered with vehicle PBS. The mice were weighed on days 0, 4, 7, 11 and 16 after administration of plasminogen, respectively. The results showed that there was no significant difference in body weight between mice in the group administered with plasminogen and those in the control group administered with vehicle PBS on days 0, 4, 7, 11 and 16 (Figure 1), indicating that plasminogen has little effect on animal body weight.

**Example 2. Protective effect of plasminogen on late hepatic tissue injury of mice with diabetic hepatic injury**

Ten male db/db mice aged 24-25 weeks were randomly divided into two groups, five in the control group administered with vehicle PBS and five in the group administered with plasminogen, respectively. The day when the experiment began was recorded on Day 0, and the mice were weighed and grouped. From the second day of the experiment, plasminogen or PBS was administered to the mice for 15 consecutive days, and the day was recorded as Day 1. Mice in the group administered with plasminogen were injected with plasminogen at a dose of 2 mg/0.2 mL/mouse/day via the tail vein, and an equal volume of PBS was administered to mice in the control group administered with vehicle PBS. Mice were sacrificed on day 16, and liver tissues were fixed in 10% neutral formalin fix solution for 24-48 hours. The fixed liver tissues were paraffin-embedded after dehydration with alcohol gradient and permeabilization with xylene. The thickness of the tissue sections was 5 $\mu$m. The sections were dewaxed and rehydrated, stained with hematoxylin and eosin (HE staining), differentiated with 1% hydrochloric acid in alcohol, and returned to blue with ammonia water. The sections were sealed after dehydration with alcohol gradient.

The HE staining results showed that in mice in the control group administered with vehicle PBS, the hepatocytes showed severe steatosis and lipid deposition, cell nuclei were squeezed to the edge, the cells showed mild hydropic degeneration, and the hepatic cord was disordered; and compared with mice in the control group administered with vehicle, in mice in the group administered with plasminogen, hepatocytes showed relieved steatosis, mild steatosis and mainly moderate hydropic degeneration. This indicated that plasminogen can promote the repair of diabetic hepatic injury.

### Example 3. Plasminogen reduces the fibrin level in liver tissues of diabetic mice

Ten male db/db mice aged 24-25 weeks were randomly divided into two groups, five in the control group administered with vehicle PBS and five in the group administered with plasminogen, respectively. The day when the experiment began was recorded on Day 0, and the mice were weighed and grouped. From the second day of the experiment, plasminogen or PBS was administered to the mice for 15 consecutive days, and the day was recorded as Day 1. Mice in the group administered with plasminogen were injected with plasminogen at a dose of 2 mg/0.2 mL/mouse/day via the tail vein, and an equal volume of PBS was administered to mice in the control group administered with vehicle PBS. Mice were sacrificed on day 16, and liver tissues were fixed in 10% neutral formalin fix solution for 24-48 hours. The fixed liver tissues were paraffin-embedded after dehydration with alcohol gradient and permeabilization with xylene. The thickness of the tissue sections was 5 $\mu$m. The sections were dewaxed and rehydrated and washed with water once. The sections were incubated with 3% hydrogen peroxide for 15 minutes and wash with water twice for 5 minutes each time. The sections were blocked with 10% normal goat serum solution (Vector laboratories, Inc., USA) for 1 hour; and after the time was up, the goat serum solution was discarded, and the tissues were circled with a PAP pen. The sections were incubated with rabbit anti-mouse fibrin (fibrinogen) antibody (Abeam) overnight at 4°C and washed with TBS twice for 5 minutes each time. The sections were incubated with a secondary antibody, goat anti-rabbit IgG (HRP) antibody (Abeam), for 1 hour at room temperature and washed with TBS twice for 5 minutes each time. The sections were developed with a DAB kit (Vector laboratories, Inc., USA). After washing with water three times, the sections were counterstained with hematoxylin for 30 seconds, flushed with running water for 5 minutes, and then washed with TBS once. After gradient dehydration, permeabilization and sealing, the sections were observed under a microscope at 200 ×.

Fibrinogen is the precursor of fibrin, and in the presence of tissue injury, as a stress response to the body's injury, fibrinogen is hydrolyzed into fibrin [12-14]. Therefore, the fibrinogen level can be used as a sign of the degree of injury.

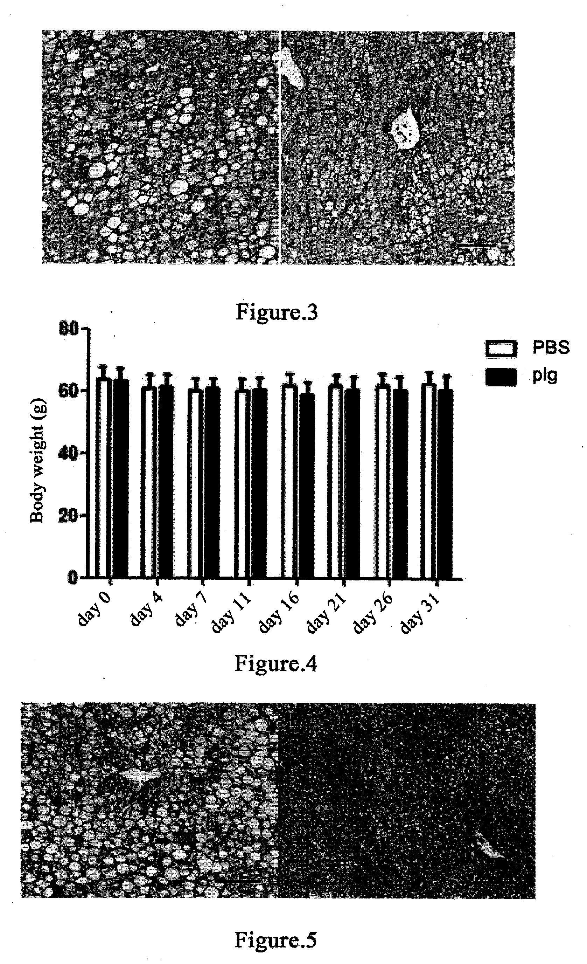

The study found that compared with mice in the control group administered with vehicle PBS (Figure 3A), those in the group administered with plasminogen (Figure 3B) had a decreased fibrin level in the liver tissues, indicating that plasminogen has the function of inhibiting the fibrin deposition and the injury is repaired to a certain degree.

### Example 4. Effect of plasminogen on body weight of diabetic mice

Twenty male db/db mice aged 24-25 weeks were randomly divided into two groups, ten in the control group administered with vehicle PBS and ten in the group administered with plasminogen, respectively. The day when the experiment began was recorded on Day 0, and the mice were weighed and grouped. From the second day of the experiment, plasminogen or PBS was administered to the mice for 31 consecutive days, and the day was recorded as Day 1. Mice in the group administered with plasminogen were injected with plasminogen at a dose of 2 mg/0.2 mL/mouse/day via the tail vein, and an equal volume of PBS was administered to mice in the control group administered with vehicle PBS. The mice were weighted on days 0, 4, 7, 11, 16, 21, 26 and 31.

The results showed that there was no significant difference in body weight between mice in the group administered with plasminogen and those in the control group administered with vehicle PBS on days 0, 4, 7, 11, 16, 21, 26 and 31 (Figure 4), indicating that plasminogen has little effect on animal body weight.

## Example 5. Protective effect of plasminogen on late hepatic tissue injury of mice with diabetic hepatic injury

Ten male db/db mice aged 24-25 weeks were randomly divided into two groups, five in the control group administered with vehicle PBS and five in the group administered with plasminogen, respectively. The day when the experiment began was recorded on Day 0, and the mice were weighed and grouped. From the second day of the experiment, plasminogen or PBS was administered to the mice for 31 consecutive days, and the day was recorded as Day 1. Mice in the group administered with plasminogen were injected with plasminogen at a dose of 2 mg/0.2 mL/mouse/day via the tail vein, and an equal volume of PBS was administered to mice in the control group administered with vehicle PBS. Mice were sacrificed on day 32, and liver tissues were fixed in 10% neutral formalin fix solution for 24-48 hours. The fixed liver tissues were paraffin-embedded after dehydration with alcohol gradient and permeabilization with xylene. The thickness of the tissue sections was 5 $\mu$m. The sections were dewaxed and rehydrated, stained with hematoxylin and eosin (HE staining), differentiated with 1% hydrochloric acid in alcohol, and returned to blue with ammonia water. The sections were sealed after dehydration with alcohol gradient.

The HE staining results showed that in mice in the control group administered with vehicle PBS (Figure 5A), the liver showed severe steatosis and lipid deposition, and fusion into large fat vacuoles, cell nuclei were squeezed to the edge (□), the hepatic cord was disordered, the hepatic sinus was narrowed, and there were different numbers of inflammatory foci at the hepatic cord (↑); and in mice in the group administered with plasminogen (Figure 5B), the liver showed mild steatosis, mainly mild hydropic degeneration at the injury, and dissolved cytoplasm (□), which were mainly distributed in the area between the portal area and the central veins; the area around the portal area and the central veins were affected mildly; and at the same time, mild inflammatory cell infiltration was seen at the hepatic cord. This indicated that hepatic injury is obviously repaired after administration of plasminogen.

## Example 6. Plasminogen reduces the fibrin level in liver tissues of diabetic mice

Ten male db/db mice aged 24-25 weeks were randomly divided into two groups, five in the control group administered with vehicle PBS and five in the group administered with plasminogen, respectively. The day when the experiment began was recorded on Day 0, and the mice were weighed and grouped. From the second day of the experiment, plasminogen or PBS was administered to the mice for 31 consecutive days, and the day was recorded as Day 1. Mice in the group administered with plasminogen were injected with plasminogen at a dose of 2 mg/0.2 mL/mouse/day via the tail vein, and an equal volume of PBS was administered to mice in the control group administered with vehicle PBS. Mice were sacrificed on day 32, and liver tissues were fixed in 10% neutral formalin fix solution for 24 hours. The fixed liver tissues were paraffin-embedded after dehydration with alcohol gradient and permeabilization with xylene. The thickness of the tissue sections was 5 $\mu$m. The sections were dewaxed and rehydrated and washed with water once. The sections were incubated with 3% hydrogen peroxide for 15 minutes and wash with water twice for 5 minutes each time. The sections were blocked with 10% normal goat serum solution (Vector laboratories, Inc., USA) for 1 hour; and after the time was up, the goat serum solution was discarded, and the tissues were circled with a PAP pen. The sections were incubated with rabbit anti-mouse fibrin (fibrinogen) antibody (Abeam) overnight at 4°C and washed with TBS twice for 5 minutes each time. The sections were incubated with a secondary antibody, goat anti-rabbit IgG (HRP) antibody (Abeam), for 1 hour at room temperature and washed with TBS twice for 5 minutes each time. The sections were developed with a DAB kit (Vector laboratories, Inc., USA). After washing with water three times, the sections were counterstained with hematoxylin for 30 seconds and flushed with running water for 5 minutes. After gradient dehydration, permeabilization and sealing, the sections were observed under a microscope at 200 ×.

Fibrinogen is the precursor of fibrin, and in the presence of tissue injury, as a stress response to the body's injury, fibrinogen is hydrolyzed into fibrin [12-14]. Therefore, the fibrinogen level can be used as a sign of the degree of injury.

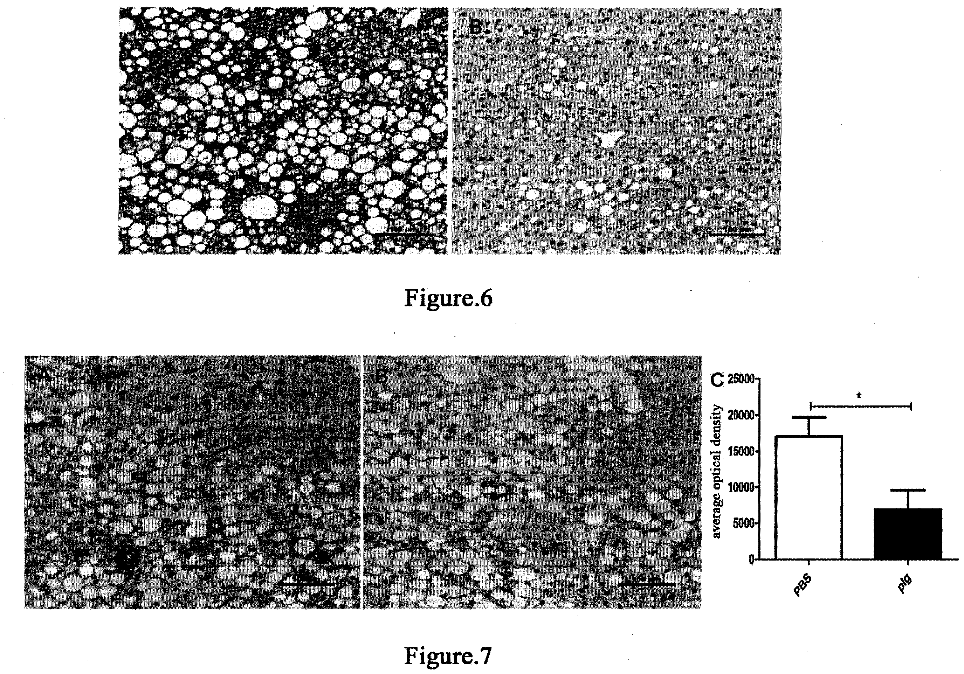

The study found that compared with mice in the control group administered with vehicle PBS (Figure 6A), those in the group administered with plasminogen (Figure 6B) had a remarkably lower fibrin level in the liver tissue, indicating that injection of plasminogen can significantly reduce the deposition of fibrin in diabetic mice, reflecting the significant repair function of plasminogen on the body's injury of diabetic mice.

## Example 7. Plasminogen reduces inflammation of liver tissues of diabetic mice

Ten male db/db mice aged 24-25 weeks were randomly divided into two groups, five in the control group administered with vehicle PBS and five in the group administered with plasminogen, respectively. The day when the experiment began was recorded on Day 0, and the mice were weighed and grouped. From the second day of the experiment, plasminogen or PBS was administered to the mice for 31 consecutive days, and the day was recorded as Day 1. Mice in the group administered with plasminogen were injected with plasminogen at a dose of 2 mg/0.2 mL/mouse/day via the tail vein, and an equal volume of PBS was administered to mice in the control group administered with vehicle PBS. Mice were sacrificed 31 days after administration of plasminogen, and liver tissues were fixed in 10% neutral formalin fix solution for 24 hours. The fixed liver tissues were paraffin-embedded after dehydration with alcohol gradient and permeabilization with xylene. The thickness of the tissue sections was 5 μm. The sections were dewaxed and rehydrated and washed with water once. The sections were incubated with 3% hydrogen peroxide for 15 minutes and wash with water twice for 5 minutes each time. The sections were blocked with 10% normal goat serum for 1 hour, and after the time was up, the serum was thrown away, and the tissues were circled with a PAP pen. The sections were incubated with a rabbit polyclonal antibody against F4/80 (Abeam) overnight at 4°C and washed with TBS twice for 5 minutes each time. The sections were incubated with a secondary antibody, goat anti-rabbit IgG (HRP) antibody (Abeam), for 1 hour at room temperature and washed with TBS twice. The sections were developed with a DAB kit (Vector laboratories, Inc., USA). After washing with water three times, the sections were counterstained with hematoxylin for 30 seconds and flushed with running water for 5 minutes. After gradient dehydration, permeabilization and sealing, the sections were observed under a microscope at 400 ×.

F4/80 is a macrophage marker that can indicate the extent and stage of an inflammatory response. The results showed that compared with mice in the control group administered with vehicle PBS (Figure 7A), the F4/80 positive level was significantly reduced in those in the group administered with plasminogen (Figure 7B), indicating that inflammation of the liver tissues is reduced after administration of plasminogen. Figure 7C shows the results of quantitative analysis of F4/80 immunohistochemical positive expression, in which the expression of F4/80 in mice in the group administered with plasminogen was significantly reduced with statistical difference, indicating that injection of plasminogen can significantly promote the repair of liver inflammation of diabetic mice.

## Example 8. Plasminogen promotes the repair of liver injury of diabetic mice

Nine male db/db mice aged 25-28 weeks were randomly divided into two groups, three in the control group administered with vehicle PBS and six in the group administered with plasminogen, respectively. The day when the experiment began was recorded on Day 0, and the mice were weighed and grouped. From the second day of the experiment, plasminogen or PBS was administered to the mice for 31 consecutive days, and the day was recorded as Day 1. Mice in the group administered with plasminogen were injected with plasminogen at a dose of 2 mg/0.2 mL/mouse/day via the tail vein, and an equal volume of PBS was administered to mice in the control group administered with vehicle PBS. Whole blood was taken from the removed eyeballs 31 days after administration of plasminogen. After the serum was precipitated, it was centrifuged at 3500 r/min for 10 minutes at 4°C, and the supernatant was taken for detection. In this experiment, the content of alanine transaminase (ALT) in serum was detected by Reitman-Frankel colorimetry using an alanine transaminase detection kit (Nanjing Jiancheng Biological Engineering Research Institute, Catalog No. C009-2).

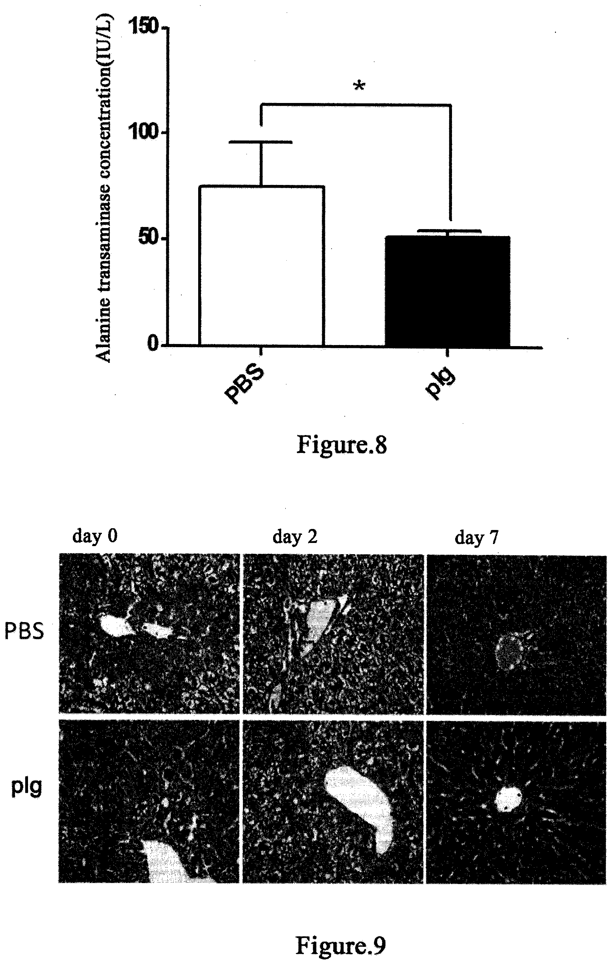

Alanine transaminase is an important index of liver health status [15,16], and the normal reference value interval of alanine transaminase is 9-50 U/L. The detection results showed that the ALT content in serum of mice in the control group administered with vehicle PBS was significantly higher than the normal physiological index, whereas the content in mice in the group administered with plasminogen had returned to normal levels in the body; and the content in mice in the group administered with plasminogen was significantly lower than that in mice in the control group administered with vehicle PBS, and there was a statistical difference (Figure 8). This indicated that injection of plasminogen can effectively repair the liver injury in model mice with late diabetic diabetes.

## Example 9. Protective effect of plasminogen on the liver in case of acute hepatic poisoning

Eighteen male or female 7-8-week-old plg+/+ mice were randomly divided into two groups, nine in the control group administered with vehicle PBS and nine in the group administered with plasminogen, respectively. Two groups of mice were administered with carbon tetrachloride via intraperitoneal injection at 0.5 mL/kg body weight for 2 consecutive days to establish an acute hepatic injury model [17,18]. Carbon tetrachloride should be diluted with corn oil before use, and the volume ratio of the former to the latter is 1 : 7. The day of model establishment was day 0, and plasminogen or PBS was administered to the mice from day 1. Mice in the group administered with plasminogen were administered with plasminogen at a dosage of 1 mg/0.1 mL/mouse/day, and an equal volume of PBS was administered to mice in the control group administered with vehicle PBS, both for 7 consecutive days. Three mice from both groups were respectively sacrificed on days 0, 2 and 7, the mice were dissected and the liver conditions were observed and recorded, and then liver tissues were fixed in 10% neutral formalin fix solution for 24-48 hours. The fixed liver tissues were paraffin-embedded after dehydration with alcohol gradient and permeabilization with xylene. The thickness of the tissue sections was 5 μm. The sections were dewaxed and rehydrated, stained with hematoxylin and eosin (HE staining), differentiated with 1% hydrochloric acid in alcohol, and returned to blue with ammonia water. The sections were sealed after dehydration with alcohol gradient and observed under a microscope at 200 ×.

The HE staining results showed that on day 0, the livers of mice in the control group administered with vehicle PBS (Figures 9A-C) and the group administered with plasminogen (Figures 9D-F) mainly showed fragmented necrosis around the central veins, in which cell nuclei were fragmented and cytoplasm was stained lightly in the necrotic area, and there were moderate hydropic degeneration and cellular edema also occurring in other non-necrotic areas. On day 2, the central veins expanded, the structures of hepatocytes were disordered, and there was a little inflammatory cell infiltration. There was no significant difference between two groups. However, on day 7, mice in the control group administered with vehicle PBS still had a little hepatocyte degeneration, mild cellular edema, disordered hepatic cord and narrowed hepatic sinus, and there was mild inflammatory cell infiltration around the portal area; whereas the liver of mice in the group administered with plasminogen had basically returned to normal cytoplasm red staining, and had regular hepatic cord and clear hepatic sinus. This indicated that plasminogen can promote the repair of hepatic injury.

### Example 10. Protective effect of plasminogen on the liver in case of acute hepatic poisoning

Eighteen male 7-11-week-old plg$^{-/-}$ mice were randomly divided into two groups, nine in the control group administered with vehicle PBS and nine in the group administered with plasminogen, respectively. Two groups of mice were administered with carbon tetrachloride via intraperitoneal injection at 0.5 mL/kg body weight once to establish an acute hepatic injury model [17,18]. Carbon tetrachloride should be diluted with corn oil before use, and the volume ratio of the former to the latter is 1 : 7. Plasminogen or vehicle PBS was administered to the mice within half an hour after completion of model establishment. Mice in the group administered with plasminogen were injected with plasminogen at a dosage of 1 mg/0.1 mL/mouse/day, and an equal volume of PBS was administered to mice in the control group administered with vehicle PBS, both for 2 consecutive days. Three mice from both groups were respectively sacrificed at hours 18, 24 and 48 after administration, the mice were dissected and the liver conditions were observed and recorded, and then liver tissues were fixed in 10% neutral formalin fix solution for 24-48 hours. The fixed liver tissues were paraffin-embedded after dehydration with alcohol gradient and permeabilization with xylene. The thickness of the tissue sections was 5 μm. The sections were dewaxed and rehydrated, stained with hematoxylin and eosin (HE staining), differentiated with 1% hydrochloric acid in alcohol, and returned to blue with ammonia water. The sections were sealed after dehydration with alcohol gradient and observed under a microscope at 200 ×.

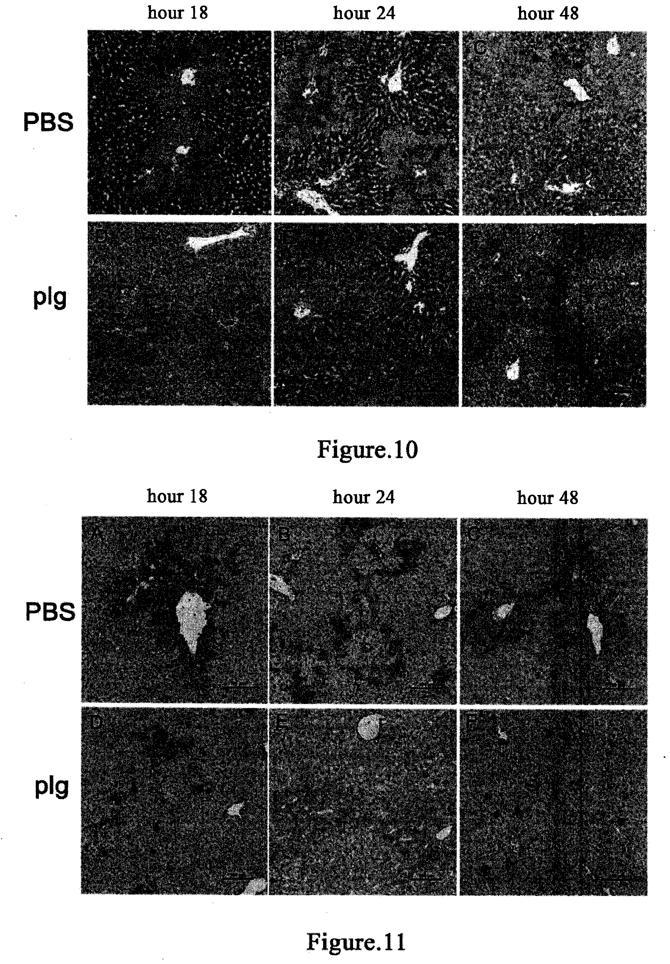

The results showed that mice in the control group administered with vehicle PBS (Figures 10A-C) showed different levels of necrosis at 18 h, 24 h and 48 h, and mainly fragmented necrosis at 18 h and 24 h, and bridging necrosis, nuclear fragmentation and lightly stained cytoplasm occurring at 48 h; the injury was progressively worsening and mainly around the central veins; and there was moderate inflammatory cell infiltration in the necrotic area (↓), and there was mild necrosis, mainly mild hydropic degeneration around the portal area, accompanied by mild inflammatory cell infiltration and mild bile duct hyperplasia (□); and compared with mice in the control group, the mice in the group administered with plasminogen (Figures 10D-F) showed no apparent necrosis at 18 h, 24 h and 48 h; the injury was mainly mild hydropic degeneration and distributed around the portal area; hepatocytes around the central veins were not affected; and these conditions were better at 24 h than those at 18 h, in which hydropic degeneration was reduced, hepatocytes around the central veins showed mild steatosis and lightly stained cytoplasm, both accompanied by mild inflammatory cell infiltration. This indicated that plasminogen can promote the repair of hepatic injury of plg$^{-/-}$ model mice with acute hepatic injury.

### Example 11. Plasminogen reduces fibrin deposition in the liver tissues of model mice with acute hepatic injury

Eighteen male 7-11-week-old plg$^{-/-}$ mice were randomly divided into two groups, nine in the control group administered with vehicle PBS and nine in the group administered with plasminogen, respectively. Two groups of mice were administered with carbon tetrachloride via intraperitoneal injection at 0.5 mL/kg body weight once to establish an acute hepatic injury model [17,18]. Carbon tetrachloride should be diluted with corn oil before use, and the volume ratio of the former to the latter is 1 : 7. Plasminogen or vehicle PBS was administered to the mice within half an hour after completion of model establishment. Mice in the group administered with plasminogen were injected with plasminogen at a dosage of 1 mg/0.1 mL/mouse/day, and an equal volume of PBS was administered to mice in the control group administered with vehicle PBS, both for 2 consecutive days. Three mice from both groups were respectively sacrificed at hours 18, 24 and 48 after administration, the mice were dissected and the liver conditions were observed and recorded, and then liver tissues were fixed in 10% neutral formalin fix solution for 24-48 hours. The fixed liver tissues were paraffin-embedded after dehydration with alcohol gradient and permeabilization with xylene. The thickness of the tissue sections was 5 μm. The sections were dewaxed and rehydrated and washed with water once. The sections were incubated with 3% hydrogen peroxide for 15 minutes and wash with water twice for 5 minutes each time. The sections were blocked with 10% normal goat serum solution (Vector laboratories, Inc., USA) for 1 hour; and after the time was up, the goat serum solution was discarded, and the tissues were circled with a PAP pen. The sections were incubated with rabbit anti-mouse fibrin (fibrinogen) antibody (Abeam) overnight at 4°C and washed with TBS twice for 5 minutes each time. The sections were incubated with a secondary antibody, goat anti-rabbit IgG (HRP) antibody (Abeam), for 1 hour at room temperature and washed with TBS twice for 5 minutes each time. The sections were developed with a DAB kit (Vector laboratories, Inc., USA). After washing with water three times, the sections were counterstained with hematoxylin for 30 seconds and flushed with running water for 5 minutes. After gradient dehydration, permeabilization and sealing, the sections were observed under a microscope at 200 ×.

Fibrinogen is the precursor of fibrin, and in the presence of tissue injury, as a stress response to the body's injury, fibrinogen is hydrolyzed into fibrin [12-14]. Therefore, the fibrinogen level can be used as a sign of the degree of injury.

The results showed that at three time points, 18 h, 24 h and 48 h, mice in the group administered with plasminogen (Figures 11D-F) showed significantly lighter fibrin-positive staining than that in mice in the control group administered with vehicle PBS (Figures 11A-C), and the fibrin staining also tended to become lighter gradually with the extension of time. This indicated that injection of plasminogen can reduce fibrin deposition and promote the repair of hepatic injury.

## Example 12. Plasminogen promotes the repair of liver inflammation of mice irradiated with 5.0 Gy X-rays

In this experiment, ten healthy 6-8-week-old male C57 mice were used and randomly divided into two groups, five in the control group administered with vehicle PBS and five in the group administered with plasminogen, respectively. After the grouping was completed, a radiation-induced injury model was established by uniformly irradiating the mice with 6 MV X-rays from a linear accelerator at 5.0 Gy once systemically, in which the absorbed dosage rate was 2.0 Gy/min and the absorbed dosage was 5.0 Gy (irradiation for 2.5 minutes). Plasminogen was administered to the mice within 3 hours after the model was established. The day when the experiment began was Day 0, and the mice were weighed and grouped. The mice were treated with radiation and administered with plasminogen or vehicle PBS from day 1. The administration period was 10 days. After the completion of the administration, the medication of animals was discontinued and they were observed for 11 days. The entire experimental period was 21 days. Mice in the group administered with plasminogen were injected with plasminogen at a dosage of 1 mg/0.1 mL/mouse/day via the tail vein, and an equal volume of PBS was administered to mice in the control group administered with vehicle PBS. Mice were sacrificed and dissected on day 21, and livers were fixed in 10% neutral formalin fix solution for 24-48 hours. The fixed liver tissues were paraffin-embedded after dehydration with alcohol gradient and permeabilization with xylene. The thickness of the tissue sections was 5 $\mu$m. The sections were dewaxed and rehydrated and washed with water once. The sections were repaired with Tris-EDTA for 30 minutes, and gently rinsed with water after cooling at room temperature for 20 minutes. The sections were incubated with 3% hydrogen peroxide for 15 minutes, and the tissues were circled with a PAP pen. The sections were blocked with 10% normal goat serum (Vector laboratories, Inc., USA) for 1 hour; and after the time was up, the goat serum solution was discarded. The sections were incubated with rabbit anti-mouse F4/80 antibody (Abeam) at 4°C overnight and washed with TBS twice for 5 minutes each time. The sections were incubated with a secondary antibody, goat anti-rabbit IgG (HRP) antibody (Abeam), for 1 hour at room temperature and washed with TBS twice for 5 minutes each time. The sections were developed with a DAB kit (Vector laboratories, Inc., USA). After washing with water three times, the sections were counterstained with hematoxylin for 30 seconds and flushed with running water for 5 minutes. After gradient dehydration, permeabilization and sealing, the sections were observed under a microscope at 200 ×.

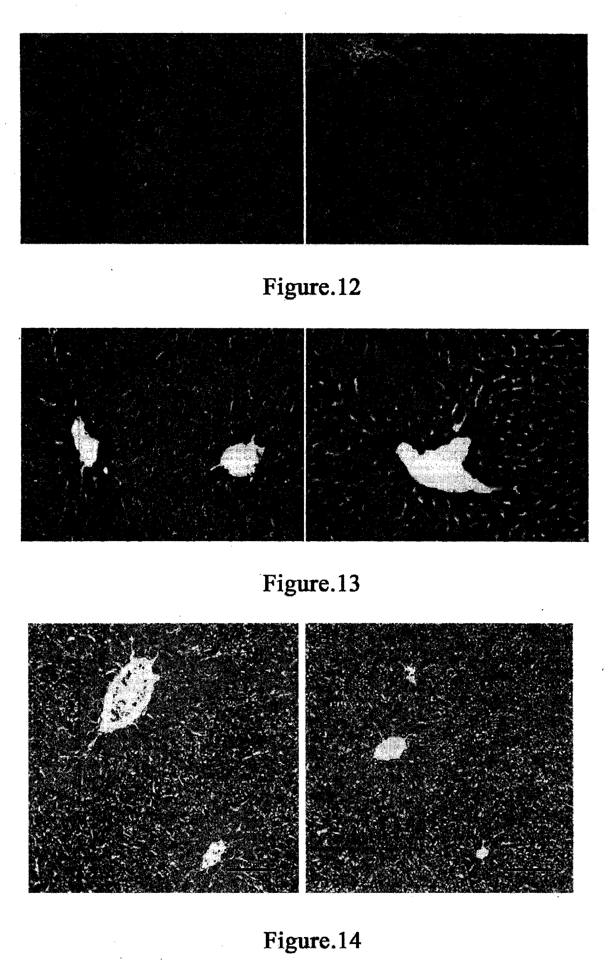

The F4/80 immunohistochemical results showed that after model establishment with 5.0 Gy X-ray irradiation, the expression level of the macrophage marker in mice in the control group administered with vehicle PBS (Figure 12A) was higher than that of the macrophage marker in mice in the group administered with plasminogen (Figure 12B), indicating that the inflammation of the liver tissues of the animals was significantly reduced after administration of plasminogen.

## Example 13. Plasminogen reduces fibrin deposition in the liver tissues of model mice with injury induced by cisplatin chemotherapy

Ten healthy 8-9-week-old male C57 mice were used and randomly divided into two groups, five in the control group administered with vehicle PBS and five in the group administered with plasminogen, respectively. After the grouping was completed, a chemotherapy-induced injury model was established by single intraperitoneal injection of cisplatin at 10 mg/Kg body weight. After the model was established, mice in the group administered with plasminogen were administered with plasminogen at a dosage of 1 mg/mouse/day via tail vein injection, and an equal volume of PBS was administered to mice in the control group administered with vehicle PBS. The day when the experiment began was Day 0, and the mice were weighed and grouped. The mice were injected with cisplatin intraperitoneally from day 1 for model establishment. Plasminogen or vehicle PBS was administered to the mice within 3 hours after completion of model establishment, and the administration period was 7 days. Mice were sacrificed on day 8, and livers were fixed in 10% neutral formalin fix solution for 24-48 hours. The fixed liver tissues were paraffin-embedded after dehydration with alcohol gradient and permeabilization with xylene. The thickness of the tissue sections was 5 $\mu$m. The sections were dewaxed and rehydrated and washed with water once. The sections were repaired with citric acid for 30 minutes, and gently rinsed with water after cooling at room temperature for 10 minutes. The sections were incubated with 3% hydrogen peroxide for 15 minutes, and the tissues were circled with a PAP pen. The sections were blocked with 10% normal goat serum (Vector laboratories, Inc., USA) for 1 hour; and after the time was up, the goat serum solution was discarded. The sections were incubated with rabbit anti-mouse fibrin antibody (Abeam) overnight at 4°C and washed with TBS twice for 5 minutes each time. The sections were incubated with a secondary antibody, goat anti-rabbit IgG (HRP) antibody (Abeam), for 1 hour at room temperature and washed with TBS twice for 5 minutes each time. The sections were developed with a DAB kit (Vector laboratories, Inc., USA). After washing with water three times, the sections were counterstained with hematoxylin for 30 seconds and flushed with running water for 5 minutes. After gradient dehydration, permeabilization and sealing, the sections were observed under a microscope at 200 ×.

Fibrinogen is the precursor of fibrin, and in the presence of tissue injury, as a stress response to the body's injury, fibrinogen is hydrolyzed into fibrin [12-14]. Therefore, the fibrinogen level can be used as a sign of the degree of injury.

The results showed that fibrin-positive staining in the liver tissues of mice in the control group administered with vehicle PBS (Figure 13A) was significantly darker than that in the liver tissues of mice in the group administered with plasminogen (Figure 13B). This indicated that plasminogen can significantly reduce fibrin deposited in injured liver tissues, indicating that plasminogen can promote the repair of hepatic injury caused by the chemotherapy drug cisplatin.

### Example 14. Protective effect of plasminogen on the liver in case of acute hepatic poisoning

Six male 7-11-week-old plg-/- mice were randomly divided into two groups, three in the control group administered with vehicle PBS and three in the group administered with plasminogen, respectively. Two groups of mice were administered with carbon tetrachloride via intraperitoneal injection at 0.5 mL/kg body weight once to establish an acute hepatic injury model [17,18]. Carbon tetrachloride should be diluted with corn oil before use, and the volume ratio of the former to the latter is 1 : 7. Plasminogen or vehicle PBS was administered to the mice within half an hour after completion of model establishment. Mice in the group administered with plasminogen were administered with plasminogen at a dosage of 1 mg/0.1 mL/mouse/day, and an equal volume of PBS was administered to mice in the control group administered with vehicle PBS, both for 7 consecutive days. Mice were sacrificed and dissected on day 8, the liver conditions were observed and recorded, and then liver tissues were fixed in 10% neutral formalin fix solution for 24-48 hours. The fixed liver tissues were paraffin-embedded after dehydration with alcohol gradient and permeabilization with xylene. The thickness of the tissue sections was 5 μm. The sections were dewaxed and rehydrated, stained with hematoxylin and eosin (HE staining), differentiated with 1% hydrochloric acid in alcohol, and returned to blue with ammonia water. The sections were sealed after dehydration with alcohol gradient and observed under a microscope at 200 ×.

The results showed that in the livers of mice in the control group administered with vehicle PBS (Figure 14A), the central veins expanded, the endothelial cells were necrotic, hepatocytes around the central veins all had large areas of focal necrosis with fragmented and darkly stained nuclei, and there were mild hydropic degeneration, cellular edema and clear cytoplasm in other non-necrotic areas, accompanied by mild inflammatory cell infiltration in the necrotic area; and in the livers of mice in the group administered with plasminogen (Figure 14B), there was no apparent necrosis, injury was mainly mild hydropic degeneration, and there were enhanced acidophily and red staining in the cytoplasm of a small amount of hepatocytes. Injury of mice in the group administered with plasminogen was obviously milder than that of mice in the control group administered with vehicle PBS, indicating that plasminogen can promote the repair of hepatic injury of plg-/- model mice with acute hepatic injury.

### References

[1] Xiao-Lan Lu, Jin-Yan Luo, Ming Tao, Yan Gen, Ping ZHAO, Hong-Li Zhao, Xiao-Dong Zhang, Nei Dong, Risk factors for alcoholic liver disease in China. World Journal of Gastroenterology. 2004, 10(16)

[2] Tim CMA Schreuder, Bart J Verwer, Carin MJ van Nieuwkerk, Chris JJ Mulder, Nonalcoholic fatty liver disease: An overview of current insights in pathogenesis, diagnosis and treatment. World Journal of Gastroenterology. 2011, 14(16)

[3] Wiman, B. and Wallen, P. (1975). Structural relationship between "glutamic acid" and "lysine" forms of human plasminogen and their interaction with the NH2-terminal activation peptide as studied by affinity chromatography. Eur. J. Biochem. 50, 489-494.

[4] Saksela, O. and Rifkin, D.B. (1988). Cell-associated plasminogen activation: regulation and physiological functions. Annu. Rev. Cell Biol. 4, 93-126

[5] Raum, D., Marcus, D., Alper, C.A., Levey, R., Taylor, P.D., and Starzl, T.E. (1980). Synthesis of human plasminogen by the liver. Science 208, 1036-1037

[6] Wallén P (1980). Biochemistry of plasminogen. In Fibrinolysis, Kline DL and Reddy KKN, eds. (Florida: CRC.

[7] Sottrup-Jensen, L., Zajdel, M., Claeys, H., Petersen, T.E., and Magnusson, S. (1975). Amino-acid sequence of activation cleavage site in plasminogen:

homology with "pro" part of prothrombin. Proc. Natl. Acad. Sci. U. S. A 72, 2577-2581.

[8] Marder V J, Novokhatny V. Direct fibrinolytic agents: biochemical attributes, preclinical foundation and clinical potential [J]. Journal of Thrombosis and Haemostasis, 2010, 8(3): 433-444.

[9] Hunt J A, Petteway Jr S R, Scuderi P, et al. Simplified recombinant plasmin: production and functional comparison of a novel thrombolytic molecule with plasma-derived plasmin [J]. Thromb Haemost, 2008, 100(3): 413-419.

[10] Sottrup-Jensen L, Claeys H, Zajdel M, et al. The primary structure of human plasminogen: Isolation of two lysine-binding fragments and one "mini"-plasminogen (MW, 38, 000) by elastase-catalyzed-specific limited proteolysis [J]. Progress in chemical fibrinolysis and thrombolysis, 1978, 3: 191-209.

[11] Nagai N, Demarsin E, Van Hoef B, et al. Recombinant human microplasmin: production and potential therapeutic properties[J]. Journal of Thrombosis and Haemostasis, 2003, 1(2): 307-313.

[12] Jae Kyu Ryu, Mark A. Petersen, Sara G. Murray et al. Blood coagulation protein fibrinogen promotes autoimmunity and demyelination via chemokine release and antigen presentation. NATURE COMMUNICATIONS,2015, 6:8164.

[13] Dimitrios Davalos , Katerina Akassoglou. Fibrinogen as a key regulator of inflammation in disease. Seminars in Immunopathology,2012. 34(1):43-62.

[14] Valvi D, Mannino DM, Mullerova H, et al. Fibrinogen, chronic obstructive pulmonary disease (COPD) and outcomes in two United States cohorts. Int J Chron Obstruct Pulmon Dis 2012;7:173-82.

[15] Karmen A, Wroblewski F, Ladue JS (Jan 1955). Transaminase activity in human blood. The Journal of Clinical Investigation. 34 (1): 126-31.

[16]Wang CS, Chang TT, Yao WJ, Wang ST, Chou P (Apr 2012). Impact of increasing alanine aminotransferase levels within normal range on incident diabetes. Journal of the Formosan Medical Association = Taiwan Yi Zhi. 111 (4): 201-8.

[17] Hua Liu, Zhe Wang, Michael J Nowicki . Caspase-12 mediates carbon tetrachloride-induced hepatocyte apoptosis in mice. World J Gastroenterol 2014 December 28; 20(48): 18189-18198.

[18] Kamyar Zahedi, Sharon L. Barone et al. Hepatocyte-specific ablation of spermine/spermidine-N1-acetyltransferase gene reduces the severity of CCl4-induced acute liver injury. Am J Physiol Gastrointest Liver Physiol 303: G546-G560, 2012.

**Claims**

1. A composition comprising an effective amount of plasminogen for use in the prevention and/or treatment of hepatic tissue injury caused by diabetes, radiation or chemical toxicants.

2. The composition for use of claim 1, wherein the plasminogen has at least 90%, 95%, 96%, 97%, 98% or 99% sequence identity with SEQ ID No.2, and still has the plasminogen activity.

3. The composition for use of claim 1 or 2, wherein the plasminogen is administered in combination with one or more other drugs.

**Patentansprüche**

1. Verbindung enthaltend eine effektive Menge Plasminogen zur Verwendung in der Vorbeugung und/oder Behandlung von hepatischen Gewebeverletzungen, die von Diabetes, Bestrahlung oder toxischen Chemikalien hervorgerufen werden.

2. Verbindung zur Verwendung nach Anspruch 1, wobei das Plasminogen mindestens 90%, 95%, 96%, 97%, 98%, oder 99% Sequenzübereinstimmung mit SEQ ID No.2 und noch die Plasminogenaktivität hat.

3. Verbindung zur Verwendung nach Anspruch 1 oder 2, wobei das Plasminogen in Kombination mit einem oder mehreren Medikamenten verabreicht wird.

**Revendications**

1. Composition comprenant une quantité efficace de plasminogène à utiliser dans la prévention et/ou le traitement des lésions du tissu hépatique causées par le diabète, les radiations ou les toxines chimiques.

2. Composition à utiliser selon la revendication 1, dans laquelle le plasminogène a au moins 90 %, 95 %, 96 %, 97 %, 98 % ou 99 % d'identité de séquence avec SEQ ID n° 2, et possède toujours l'activité plasminogène.

3. Composition à utiliser selon la revendication 1 ou 2, dans laquelle le plasminogène est administré en association avec un ou plusieurs autres médicaments.

Figure.1

Figure.2

Figure.3

Figure.4

Figure.5

Figure.6

Figure.7

Figure.8

day 0          day 2          day 7

PBS

plg

Figure.9

Figure.10

Figure.11

Figure.12

Figure.13

Figure.14

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 102154253 A

- US 3773919 A
- EP 58481 A

**Non-patent literature cited in the description**

- **BARANY.** pecial Methods in Peptide Synthesis. *The Peptides: Analysis, Synthesis, Biology,* vol. 2, 3-284
- **MERRIFIELD et al.** *J. Am. Chem. Soc.,* 1963, vol. 85, 2149-2156
- **STEWART et al.** Solid Phase Peptide Synthesis. Pierce Chem. Co, 1984
- **GANESAN A.** *Mini Rev. Med Chem.,* 2006, vol. 6, 3-10
- **CAMARERO JA et al.** *Protein Pept Lett.,* 2005, vol. 12, 723-8
- **WINNACKER.** From Genes to Clones. VCH Publishers, 1987
- **QUEEN et al.** *Immunol. Rev.,* 1986, vol. 89, 49
- **CO et al.** *J. Immunol.,* 1992, vol. 148, 1149
- Remington's Pharmaceutical Sciences. 1980
- **LANGER et al.** *J. Biomed. Mater. Res.,* 1981, vol. 15, 167-277
- **LANGER.** *Chem. Tech.,* 1982, vol. 12, 98-105
- **SIDMAN et al.** *Biopolymers,* 1983, vol. 22, 547
- **XIAO-LAN LU ; JIN-YAN LUO ; MING TAO ; YAN GEN ; PING ZHAO ; HONG-LI ZHAO ; XIAO-DONG ZHANG ; NEI DONG.** Risk factors for alcoholic liver disease in China. *World Journal of Gastroenterology,* 2004, vol. 10 (16
- **TIM CMA SCHREUDER ; BART J VERWER ; CARIN MJ VAN NIEUWKERK ; CHRIS JJ MULDER.** Nonalcoholic fatty liver disease: An overview of current insights in pathogenesis, diagnosis and treatment. *World Journal of Gastroenterology,* 2011, vol. 14 (16
- **WIMAN, B. ; WALLEN, P.** Structural relationship between "glutamic acid" and "lysine" forms of human plasminogen and their interaction with the NH2-terminal activation peptide as studied by affinity chromatography. *Eur. J. Biochem.,* 1975, vol. 50, 489-494
- **SAKSELA, O. ; RIFKIN, D.B.** Cell-associated plasminogen activation: regulation and physiological functions. *Annu. Rev. Cell Biol.,* 1988, vol. 4, 93-126

- **RAUM, D. ; MARCUS, D. ; ALPER, C.A. ; LEVEY, R. ; TAYLOR, P.D. ; STARZL, T.E.** Synthesis of human plasminogen by the liver. *Science,* 1980, vol. 208, 1036-1037
- Biochemistry of plasminogen. **WALLÉN P.** Fibrinolysis. CRC, 1980
- **SOTTRUP-JENSEN, L. ; ZAJDEL, M. ; CLAEYS, H. ; PETERSEN, T.E. ; MAGNUSSON, S.** Amino-acid sequence of activation cleavage site in plasminogen: homology with "pro" part of prothrombin. *Proc. Natl. Acad. Sci. U. S. A,* 1975, vol. 72, 2577-2581
- **MARDER V J ; NOVOKHATNY V.** Direct fibrinolytic agents: biochemical attributes, preclinical foundation and clinical potential. *Journal of Thrombosis and Haemostasis,* 2010, vol. 8 (3), 433-444
- **HUNT J A ; PETTEWAY JR S R ; SCUDERI P et al.** Simplified recombinant plasmin: production and fu-nctional comparison of a novel thrombolytic molecule with plasma-derived plasmin. *Thromb Haemost,* 2008, vol. 100 (3), 413-419
- **SOTTRUP-JENSEN L ; CLAEYS H ; ZAJDEL M et al.** The primary structure of human plasminogen: Isolation of two lysine-binding fragments and one "mini"-plasminogen (MW, 38, 000) by elastase-catalyzed-specific limited proteolysis. *Progress in chemical fibrinolysis and thrombolysis,* 1978, vol. 3, 191-209
- **NAGAI N ; DEMARSIN E ; VAN HOEF B et al.** Recombinant human microplasmin: production and potential therapeutic properties. *Journal of Thrombosis and Haemostasis,* 2003, vol. 1 (2), 307-313
- **JAE KYU RYU ; MARK A. PETERSEN ; SARA G. MURRAY et al.** Blood coagulation protein fibrinogen promotes autoimmunity and demyelination via chemokine release and antigen presentation. *NATURE COMMUNICATIONS,* 2015, vol. 6, 8164
- **DIMITRIOS DAVALOS ; KATERINA AKAS-SOGLOU.** Fibrinogen as a key regulator of inflammation in disease. *Seminars in Immunopathology,* 2012, vol. 34 (1), 43-62

- **VALVI D ; MANNINO DM ; MULLEROVA H et al.** Fibrinogen, chronic obstructive pulmonary disease (COPD) and outcomes in two United States cohorts. *Int J Chron Obstruct Pulmon Dis,* 2012, vol. 7, 173-82
- **KARMEN A ; WROBLEWSKI F ; LADUE JS.** Transaminase activity in human blood. *The Journal of Clinical Investigation,* January 1955, vol. 34 (1), 126-31
- **WANG CS ; CHANG TT ; YAO WJ ; WANG ST ; CHOU P.** Impact of increasing alanine aminotransferase levels within normal range on incident diabetes. *Journal of the Formosan Medical Association,* 2012, vol. 111 (4), 201-8
- **HUA LIU ; ZHE WANG ; MICHAEL J NOWICKI.** Caspase-12 mediates carbon tetrachloride-induced hepatocyte apoptosis in mice. *World J Gastroenterol,* 28 December 2014, vol. 20 (48), 18189-18198
- **KAMYAR ZAHEDI ; SHARON L. BARONE et al.** Hepatocyte-specific ablation of spermine/spermidine-N1-acetyltransferase gene reduces the severity of CCl4-induced acute liver injury. *Am J Physiol Gastrointest Liver Physiol,* 2012, vol. 303, G546-G560